# EUROPÄISCHE PATENTANMELDUNG

(11) **EP 2 845 581 A1**
(43) Veröffentlichungstag der Anmeldung: **11.03.2015**
(21) Anmeldenummer: 14183647.8
(22) Anmeldetag: 05.09.2014
(51) Int. Cl.: A61K 8/24, A61K 6/033, A61K 8/02

(54) **Formulierungen und Kit zur biomimetischen Abscheidung von Apatit auf Zähnen**

(30) Priorität: 09.09.2013 DE 102013109847
(71) Anmelder: Heraeus Kulzer GmbH, 63450 Hanau (DE)
(72) Erfinder: Busch, Susanne, 61267 Neu Anspach (DE); Utterodt, Andreas, 61267 Neu Anspach (DE); Gerlach, Michael, 65719 Hofheim (DE)
(74) Vertreter: Bendele, Tanja

(57) **Zusammenfassung**

Es wird ein Kit sowie Formulierungen zur biomimetischen Abscheidung von Apatit aus einem teilelastischen, eine Hülle aufweisenden Formkörper auf Zähnen vorgeschlagen, wobei der Formkörper a) mindestens eine Mineralisationsmatrix enthaltend ein Gel umfasst, das wasserlösliche Phosphate oder zu wasserlöslichen Phosphat-Ionen hydrolysierbare Phosphate und einen pH-Wert von 2 bis 8 aufweist, optional Fluoride und b) die mindestens eine oder eine zweite Mineralisationsmatrix ein zweites Gel mit einem pH-Wert von 3.5 bis 14 aufweist, umfassend Calcium-Ionen oder Calcium-lonen freisetzende Verbindungen. Darüber hinaus wird das Verfahren zur Herstellung der Formulierung, als auch deren Verwendung zur Abscheidung von Apatit beansprucht, insbesondere von nadelförmigen Fluorapatitkristalliten. Mit den erfindungsgemässen Formulierungen ist es möglich innerhalb einer Schlafperiode grösser gleich 1 µm Apatit, insbesondere Fluorapatit auf Zahnoberflächen abzuscheiden, um poröse Zahnoberflächen zu verschliessen oder aufzuhellen.

## Beschreibung

Es wird ein Kit sowie Formulierungen zur biomimetischen Abscheidung von Apatit aus einem teilelastischen, zumindest eine teilweise Hülle aufweisenden Formkörper auf Zähnen vorgeschlagen, wobei der Formkörper a) mindestens eine Mineralisationsmatrix enthaltend ein Gel umfasst, das wasserlösliche Phosphate oder zu wasserlöslichen Phosphat-Ionen hydrolysierbare Phosphate und einen pH-Wert von 2 bis 8 aufweist, optional Fluoride und b) die mindestens eine oder eine zweite Mineralisationsmatrix ein zweites Gel mit einem pH-Wert von 3.5 bis 14 aufweist, umfassend Calcium-Ionen oder Calcium-Ionen freisetzende Verbindungen. Darüber hinaus wird das Verfahren zur Herstellung der Formulierung, als auch deren Verwendung zur Abscheidung von Apatit beansprucht, insbesondere von nadelförmigen Fluorapatitkristalliten. Mit den erfindungsgemässen Formulierungen ist es möglich innerhalb einer Schlafperiode grösser gleich 1 µm Apatit, insbesondere Fluorapatit auf Zahnoberflächen abzuscheiden, um poröse Zahnoberflächen zu verschliessen oder aufzuhellen.

Zähne sind harte Biomaterialien in Form von Verbundstoffen basierend auf Proteinen und Apatit umfassend Calcium und Phosphat. Der Zahnschmelz, die äussere Schicht der Zahnkrone ist der härteste Teil des Zahns und enthält keine lebenden Zellen. Er besteht aus anorganischen Kristallen, welche typische hoch orientierte Anordnungen aufweisen. Zahnschmelz ist ein Gewebe, das, sobald es einmal gebildet ist, lebenslang nahezu unverändert bleibt, da die Zellen, welche beim Aufbau der Zähne beteiligt sind, absterben, sobald die Zahnbildung abgeschlossen ist. Ein fertiger Zahnschmelz besteht aus etwa 95 Gew.-% Apatit, etwa 3 Gew.-% Proteinen und Lipiden und etwa 2 Gew. -% Wasser.

Um Schädigungen von Zähnen, insbesondere durch Karies, zu vermeiden oder zu reparieren, wurde seit langem versucht, remineralisierende Systeme einzusetzen. Dabei wurde zunächst versucht, durch Aufbringen von Calciumphosphat-verbindungen die Beschaffenheit der Zähne zu verbessern.

Solche Einkomponentensysteme, bei denen versucht wird, bereits vorgefertigtes Zahnmaterial, beispielsweise Apatit, Hydroxyapatit oder andere Calciumphosphat-verbindungen auf die Zähne aufzubringen, sind unter anderem in EP 0 666 730 B1 oder WO 01/95863 beschrieben. Das Problem solcher Systeme besteht darin, dass das Behandeln von Zahnmaterial mit Calciumphosphat-Verbindungen nicht zu einem Aufwachsen von strukturell dem Zahnmaterial ähnlichen Apatit führt, sondern vielmehr höchstens zu einer blossen Anlagerung von Apatitkristallen auf dem Zahnmaterial, wobei die Apatitkristalle eine vom Zahnmaterial völlig unterschiedliche Morphologie aufweisen. Somit wird keine Festigung des Zahnschmelzes oder dauerhafte Füllung von Läsionen bewirkt, da die angelagerten Apatitkristalle keine ausreichende Ähnlichkeit und Haftung zum natürlichen Zahnmaterial aufweisen.

Bedingt durch moderne Ernährungsgewohnheiten die häufig saure Lebensmittel enthalten, nehmen nicht bakteriell bedingte Erosionen an der Zahnhartsubstanz zu [Dentale Erosionen: Von der Diagnose zur Therapie, Adrian Lussi, Thomas Jaeggi, Quitessenz Verlag].

Nicht nur Lebensmittel wie stark gesäuerte Süssigkeiten, Softdrinks oder Alkopops spielen hier eine Rolle, sondern auch eine zunehmend stark obsthaltige Ernährung kann zu Zahnproblemen führen. Durch die kontinuierliche Einwirkung von Säuren wird der Zahnschmelz dünner und auch poröser. In Extremfällen kann der Schmelz vollständig aufgelöst bzw. abradiert werden, das empfindliche Dentin liegt dann frei. Insbesondere im Zahnhalsbereich, der nur von einer sehr dünnen Schicht Schmelz geschützt wird, tritt dies häufig auf. Die säurebedingte Erosion kann dann noch schneller weiter voranschreiten, da Dentin säurelöslicher ist als Schmelz und es kommt häufig zu keilförmigen Defekten der Zahnhartsubstanz. Freiliegendes Dentin führt zu sensiblen, schmerzempfindlichen Zähnen. Sensible Zahnhälse können aber auch die Folge falscher Putzgewohnheiten sein. Auch zunehmendes Alter ist ein Grund für dünner werdenden Schmelz. Regelmässiges Knirschen kann den Schmelz an den Schneidekanten abradieren. Da durch verbesserte Prophylaxe in der Zahnheilkunde und die konsequente Fluoridierung der meisten Zahncremes und zusätzlicher Pflegeprodukte die Karies zurück geht, die Bevölkerung in den Industriestaaten aber immer älter wird und die funktionsfähigen Zähne länger arbeiten müssen, wächst auch die Bedeutung von nicht kariogenen Zahnhartsubstanzverlusten.

Einige Darreichungsformen, die als geeignet beschrieben werden, die Mineralisation von Apatit auf der Zahnoberfläche anzuregen, sind bekannt. U.S. Pat. No. 6,521,251 beschreibt eine Zusammensetzung, die neben Carbamidperoxid auch Calziumphosphate enthält, deren Löslichkeit etwas besser ist, als die von Apatit, wie Mono-Di-oder Trikalziumphosphat. Dennoch sind all diese Calziumphosphate nur wenig wasserlöslich, daher ist insbesondere bei den beschriebenen Zahnputzmitteln eher eine abrasive als eine remineralisierende Wirkung zu erwarten. Tatsächlich beschreibt U.S. Pat. 5,851,514 unter anderem die Zugabe von Dikalziumphosphat als Abrasivum.

U.S. Pat. No. 6,419,905 erwähnt die Zugabe von Kaliumsalzen (z. B. Citrat) und Fluorid zum Peroxid. Fluorid ist geeignet, um aus dem Speichel insbesondere Kalzium- und Phosphat-Ionen zu binden, wodurch Fluorapatit ausfällt. Wenn keine weiteren Ionen zugefügt werden, wird auch die Bildung von CaF₂ beobachtet. Die Calziumfluoridpartikel können in der Plaque gespeichert werden und über längere Zeit Fluorid abgeben, da sie löslicher sind, als der Apatit der Zahnhartsubstanz. Jedoch werden Zähne durch die gewissenhafte wiederholte tägliche Reinigung von Plaque weitgehend befreit. Damit ist die Wirkung von Calciumfluorid nur von kurzer Dauer und die fluoridhaltigen Produkte müssen regelmässig angewendet werden. Eine Bildung von neuem Apatit wurde mit solchen Produkten nicht beobachtet.

Patent JP20000051804 beschreibt den parallelen Einsatz von konzentrierter Phosphorsäure, konz. H₂O₂ und Fluorapatitpulver. Problematisch erscheint dabei der Einsatz der konzentrierten Phosphorsäure, die gesunden Zahnschmelz merklich auflösen kann. Zudem ist die Bleichlösung stark reizend und darf nicht in Kontakt mit dem Zahnfleisch kommen, was aber in abgeschwächter Form für alle oxidativ wirkenden Zahnbleichmittel gilt. Zudem führt die wiederholte Anwendung nicht zum Aufbau einer Mineralisationsschicht.

Eine säurefreie Anwendung wird in U.S. 20050281759 beschrieben. Als wesentlicher Inhaltsstoff wird hier Kalziumperoxophosphat vorgeschlagen. Kerngedanke ist hier, dass eine einzige Substanz aufhellend und remineralisierend wirken soll, da parallel zur Oxidation die Freisetzung von Kalzium- und Phosphat-Ionen ausgelöst wird. Nicht klar ist, ob die Salze in der verhältnismässig kurzen Einwirkungsdauer einen nennenswerten Apatitaufbau leisten können. U.S. Pat. No. 6,303,104 beschreibt ein oxidationsmittelfreies Zwei-Komponentensystem aus löslichen Kalzium- und Phosphatsalzen, dass auch eine aufhellende Wirkung haben soll. Die Aufhellung soll durch die Zugabe von Natriumgluconat hervorgerufen werden, welches färbende Metallionen (z. B. Eisen) aus dem Zahnschmelz komplexiert. Beim Vermischen der Komponenten ist sofort mit einer Ausfällung der schwerlöslichen Kalziumphosphate zu rechnen und es ist nicht zu erkennen, warum es zu ausgeprägter Remineralisation kommen soll, zumal es sich bei dem Produkt um eine Zahncreme handelt, die nur wenige Minuten in Kontakt mit den Zahnflächen verbleibt. U.S. Pat. 6,102,050 beschreibt eine Zahnseide mit Titandioxidpartikeln, der eine aufhellende, remineralisierende und desensibilisierende Wirkung auf die Interdentalflächen zugeschrieben wird. Es sollen Titandioxidmikropartikel der Grösse 0.1 bis 1.5 µm sowohl als mildes Abrasivum wirken, als auch vom Schmelz absorbiert werden, was mit einer aufhellenden Wirkung verbunden sei. Vermutlich können sich die Partikel allenfalls mechanisch in geeignete Hohlräume einlagern, was keine stabile Verankerung verspricht und nur einen temporären Effekt haben kann.

Alle bisher beschriebenen Patente berücksichtigen nicht, dass Biominerale ihre hohe strukturelle Organisation und Stabilität nur erreichen, weil sie sich in Gegenwart von speziellen Biomolekülen bilden, die Mikro- und Makrostrukturierung vorgeben.

WO 2005/027863 beschreibt ein Zahnpflegemittel, dass über reinigende, remineralisierende, desensibilisierende und aufhellende Wirkung verfügen soll. Als aktive Komponente zur Remineralisation und Aufhellung wird ein nanoskaliges Apatit-Gelatine-Komposit vorgeschlagen, das in Gegenwart einer wässrigen Gelatinelösung gefällt wird und daher Polypeptide eingelagert hat. Dieses Material soll durch sogenannte "Neomineralisation" auf der Zahnoberfläche einen Schutzfilm von dentinähnlicher Struktur bilden können, der eine Oberflächenglättung bewirken soll und offene Dentintubuli verschliessen können soll. Diese Wirkung ist für eine Zahncreme nicht nachvollziehbar, da bevorzugt nur 0.01-2 Gew.-% "Nanite" (WO 01/01930) in diesem Zahnpflegemittel enthalten sind. Die Einwirkung der aktiven Substanzen liegt bei nur maximal einigen Minuten täglich. Eine ausgeprägte oder flächendeckende Mineralabscheidung wird bezweifelt. Eine Mineralabscheidung auf Schmelz ist zudem nicht beschrieben. Ein kontinuierlicher Zuwachs der Filmdicke bei längerer Anwendung des Pflegemittels wird ebenfalls nicht erwähnt. Zudem ist die poröse, wenig geordnete Struktur von Dentin nicht in der Lage, den Zahn vor korrosiven Angriffen zu schützen. Das kommerziell erhältliche Produkt Tooth Mousse oder Mi-Paste basiert auf Patentschriften von Reynolds [WO 98/40406] und soll porösen Schmelz remineralisieren. Die Erfindung beruht darauf, dass Casein (CPP) eine stabilisierende Wirkung auf amorphes Calciumphosphat (ACP) besitzt. Der Wirkstoff CPP-ACP soll in Kontakt mit Zahnhartsubstanz zu Hydroxylapatit remineralisieren. Ein solcher Schutzfilm von dentinartiger Struktur erscheint nicht geeignet, um einen dauerhaften Schutz zu bieten.

Allen Patenten ist gemein, dass sie nur von einer Remineralisation sprechen ohne diese zu belegen und/oder desensibilisierende Wirkung haben. US 2012/0027829A1 beschreibt die Bildung von Hydroxylapatitschichten (HAP) auf Dentin, wenn wiederholt pastenförmige Mischungen aus Propylenglykol, Glycerin, Xylitol, Polyethylenglykol, Cethylpyridiniumchlorid, Tetracalciumphosphat und ein Alkalisalz der Phosphorsäure auf die Zähne aufgebracht werden. Da Tetracalciumphosphat in Gegenwart von Wasser sofort mit den Salzen der Phosphorsäure reagiert, werden zuvor zwei getrennte Pasten hergestellt die erst kurz von der Anwendung gemischt werden. Eine Bildung von HAP auf Schmelz wird nicht beschrieben und es gibt keine Daten zur gebildeten Schicht, die bei eigenen Versuchen auch nicht nachstellbar war.

Die Möglichkeit einer, bei wiederholter Anwendung immer dicker werdenden Fluorapatit-Schicht mit Zahnschmelz-ähnlicher Festigkeit bietet die in US2005220724 und DE 10 2004 054 584.7 beschriebene Technik. Wasserlösliche Phospat- und Fluorid-Salze werden in dem gepufferten Gel A, Kalzium-Ionen im Gel B eingearbeitet. Optional durch eine ionenfreie Schutzschicht getrennt, werden die bei physiologischer Temperatur festen Gelatine-Gele unter Erwärmung nacheinander auf Zahnoberflächen aufgetragen. In Abhängigkeit von den Austauschzyklen der Gele kann eine Zunahme der Schichtstärke beobachtet werden. Die Wachtumsgeschwindigkeiten betragen 0.5 bis 5.0 µm/Tag. Die biologischen Strukturen des Zahnmaterials werden individuell vom Fluorapatit abgebildet, Hohlräume durch offenliegende Dentintubuli werden nach einigen Austauschzyklen verschlossen.

Hinsichtlich einer Anwendung am Menschen erweist es sich als ungünstig, dass die Gele vor der Applikation erwärmt werden müssen. Durch das Aufbringen der zweiten und dritten Gelschicht können darunter liegende, bereits applizierte Gelschichten wieder verflüssigt werden und sich in unerwünschter Weise mit darüber liegenden Schichten vermischen. Insbesondere kleinere Darreichungsmengen trocknen bei Exposition an der Luft schnell aus und ein Verflüssigen durch Erwärmung ist dann nicht mehr ohne weiteres möglich. Die Methode erlaubt kaum, genau definierte Gelmengen gleicher Dicke auf den Zahn aufzubringen. Ferner tragen die drei bis zu 6 mm dicken Gelschichten stark auf, was bei Schutzsystemen wie Schienen oder Pflastern zu Problemen führt, da hier Platz für grosse Gelreservoirs geschaffen werden muss.

Ferner wird die Methode zunehmend aufwändig, wenn der gesamte Kiefer mit allen Zahnflächen behandelt werden soll. Da zur Bildung von Fluorapatit idealer Weise eine Anwendungsdauer von 8 Stunden nicht unterschritten werden sollte, wäre es von Vorteil, wenn der Patient das System selbst anwenden könnte, indem er es vor dem Schlafengehen anwendet. Dazu müsste er die Gele erwärmen und präzise auf den Zähnen platzieren, was sehr schwer möglich ist, da erwärmte flüssige Gelatine sehr klebrig ist. Zudem ist die Verbrennungsgefahr gross. Ein weiterer Nachteil ist, dass die Gele im Mundmilieu flüssig bleiben und nicht sicher auf den Zähnen haften.

Da die Gele trotz eines Schutzes wie z.B. einer individualisierten Tiefziehschiene auslaufen, muss die Schiene mit einem geeigneten Abdichtsystem abgedichtet werden, was die Methode noch komplizierter macht.

Die Verwendung von vorgefertigten Gelstreifen DE 10 2006 055 223 A1 hat den Vorteil, dass das aufwändige Erwärmen entfällt und die Streifen die gleiche Dicke haben. Ein grosser Nachteil ist allerdings, dass die Streifen nur Teilareale der Zähne erreichen. Da Erosionen aber mehr oder weniger alle Flächen der Zähne betreffen, wäre es wünschenswert, dass das Mineralisations-Kit auch überall wirken kann. Ferner ist es sehr umständlich die zwei Streifen auszupacken und beispielsweise in eine Tiefziehschiene einzubringen, die überdies noch ein Reservoir für die Gelstreifen haben muss..Das Problem der Abdichtung der Schienen ist nicht zufriedenstellend gelöst. Da die Streifen sich unter Mundmilieu verflüssigen, ist eine Abdichtung aber notwendig, da die Wirkstoffe sonst in den Mundraum auslaufen können.

Die Erfindung beschreibt eine Methode basierend auf den Patenten US2012195941 (A1) und US2008241797 (A1) mit der eine schmelzähnliche Schicht aus Fluorapatit auf Zähne aufgebracht werden kann. In der beschriebenen Ausführungsform sind zwei Mineralisationsstreifen so geformt, dass einzelne Zahnflächen, eine Gruppe von Zähnen oder mehrere Zahnflächen auf einmal behandelt werden können. Möglich ist auch eine Behandlung von Zahnflächen ganzer Kiefer.

Hintergrund dieser Erfindung war das Ziel einen Mineralisations-Kit anbieten zu können, der mit hoher Reproduzierbarkeit qualitativ hochwertige zahnschmelzähnliche Überzüge aus Apatit auf Zahnhartsubstanz erzeugt mit dem Ziel diesen vor übermässigem Zahnschmelzverlust zu schützen. Die Inhaltsstoffe sollen weitgehend dem biologischen Vorbild entsprechen und die Anwendung soll möglichst einfach sein. Weitere Aufgaben waren die Anwendung deutlich zu vereinfachen, vorzugsweise soll eine Anwendung sowohl beim Zahnarzt als auch direkt durch den Anwender möglich sein. Zudem soll die Anwendungsdauer der einzelnen Anwendung eines Kits erhöht werden. Daher bestand eine Aufgabe, die Problematik der Abdichtung der Gele zu lösen und eine Formulierung bereitzustellen, in der vorzugsweise auf eine separate Abdichtung verzichtet werden kann, ohne die Abscheidung des Apatits zu verhindern.

Gelöst werden die Aufgaben durch eine Formulierung nach Anspruch 1 und ein Kit nach Anspruch 30 sowie durch die Verfahren zur Herstellung der Formulierung nach den Ansprüchen 23 und 24.

Die Aufgaben wurden gelöst indem die auf Basis von denaturiertem Kollagen oder anderen Gelbildnern und Mineralstoffen zusammengesetzte Mineralisationsmatrix durch chemische Modifikation im Mundmilieu unlöslich wird, insbesondere als eine Formulierung mit getrennten Formkörpern oder als mehrteiliger Formkörper umfassend jeweils mindestens eine oder zwei Mineralisaitionsmatrices. Überraschend wird die Mineralisationsaktivität trotz der Modifizierung positiv beeinflusst, da das Gel im Mundmilieu nicht mehr verläuft und während der gesamten Behandlungsdauer wirken kann. Diese chemische Modifikation erfolgt vorzugsweise durch zumindest teilweise oberflächliche chemische Vernetzung der Mineralisationsmatrix, so dass mindestens eine teilweise chemisch vernetzte Ebene oder teilweise Hülle gebildet wird. Vorzugsweise ist die mindestens eine Ebene eine Oberfläche der Mineralisationsmatrix, bevorzugt bilden die Ebenen eine äussere Hülle an den Oberflächen der Mineralisationsmatrix aus. Erfindungsgemäss wirken die vernetzten Ebenen oder die Hülle wie eine Membran, durch die wässrige Medien, wie Speichel, in die Mineralisationsmatrix eindringen können und zugleich eine Abscheidung von Apatit ausserhalb der Mineralisationsmatrix auf den Zahnoberflächen erfolgen kann, die mit der mindestens einen Ebene oder der Hülle der Mineralisationsmatrix in Kontakt ist. Die gebildete Ebene oder Hülle kann sich erfindungsgemäss einerseits durch ihre sehr dünne Schicht optimal bei einer Quellung im Mund an die Oberflächen der Zähne anlegen und begünstigt auch die Abscheidung von Apatit im Bereich der Zahnzwischenräume. Ferner legt sich die Minearilsationsmatrix bei Körpertemperatur optimal an die Zahnkontur an. Ein weiterer besonderer Vorteil der oberflächlichen Vernetzung der Mineralisationsmatrix, bei der sich ein Formkörper ausbildet, ist, dass ein zumindest teilelastischer Formkörper gebildet wird, der sich optimal der Oberflächenkontur von Zähnen und Zahnreihen anschmiegen kann. Besonders gut liegt der teilelastische Formkörper durch die Quellung der Mineralisationsmatrix im Mundmilieu an den Zahnoberflächen an. Um den Formkörper, umfassend mindestens eine Mineralisationsmatrix, optimal an den bukkalen, labialen, mesialen und/oder approximalen Oberflächen der Zähne zu fixieren, wird vorteilhaft der mindestens eine Formkörper in eine Zahnschiene eingelegt bzw. in der Zahnschiene bereitgestellt. Durch eine Fixierung mit einer Zahnschiene gelingt es mit dem erfindungsgemässen Formkörper auch, auf mindestens einem Teil oder nahezu vollständig auf den Zähnen sowohl des Ober- als auch des Unterkiefers bukkal, mesial, labial, palatinal, distal sowie approximal Apatit abzuscheiden. Durch den erfindungsgemässen mindestens einen Formkörper kann auf einfache Weise erstmalig einfach durch Einsetzen der Formkörper in eine Schiene und Aufsetzen der Schiene auf die Zähne über Nacht bspw. etwa 8 bis 12 Stunden, optional bis 16 oder 24 Stunden, alternativ auch am Tag eine biomimetische Remineralisierung von mindestens teilweise grösser gleich 1 µm erfolgen, bevorzugt grösser gleich 2 µm, bevorzugt grösser gleich 3 µm, besonders bevorzugt ist eine biomimetische Reminerasilierung von mindestens teilweise, vorzugweise im Mittel, von 1 bis 10 µm, 1 bis 5 µm. Besonders bevorzugt erfolgt die Remineralisierung in der Fläche mit einer möglichst homogen Schichtdicke.

Überraschender Weise wurde festgestellt, dass sich das Mineralisationsprodukt nach der chemischen Stabilisierung gleichmässiger und dichter auf der Zahnoberfläche bildet. Die vernetzte Ebene oder Hülle ist so porös, dass trotz Ausbildung der Hülle vergleichbare Apatitschichten abgeschieden werden können, wie die erfindungsgemässen Beispiele 2 bis 4 und 6 gegenüber den Beispielen 1 und 5 im Vergleich aufzeigen. Es wird davon ausgegangen, dass sich durch die chemische Modifizierung ein weniger temperaturempfindliches und vorzugsweise weniger hydrolyseempfindliches Netz aus Polypeptiden um die Mineralisationsmatrix bildet, dessen Poren jedoch noch gross genug sind, um Moleküle und Ionen durchzulassen, welche die stabile, geordnete Apatitschicht an der Zahnoberfläche bilden. Die chemisch vernetzte Schicht oder Ebene wirkt quasi wie eine Ionen und moleküldurchlässige Membran.

Eine ausreichend dicke, homogene und stabile Apatitschicht lässt sich durch das optimale Zusammenspiel der Komponenten: Mineralsalze, Puffer und pH-Wert erzielen. Ausschlaggebend sind die korrekt gewählten Konzentrationen bei entsprechendem Vernetzungsgrad. Die erfindungsgemässe Formulierung kann zur Abscheidung von Apatit auf Zähnen oder jeglichen anderen biologischen Oberflächen wie auf einer Knochenmatrix eingesetzt werden.

Die einfachere Anwendung der erfindungsgemässen Formulierungen eröffnet die Möglichkeit, dass sowohl Zahnärzte an Patienten als auch Patienten selbst das System anwenden können. Die Abscheidung einer fluoridreichen Calciumphosphatschicht kann Sensibilitäten an den Zähnen vermindern, sie kann Risse und initiale Porösitäten reduzieren und erhöht die Säurestabilität der Zähne. Initiale Zahnhartsubstanzverluste infolge von Säureerosion können gestoppt bzw. teilweise oder vollständig rückgängig gemacht werden. Der erhöhte Fluoridgehalt im Vergleich zu unbehandelten Zähnen in den Schichten reduziert die Löslichkeit des neu gebildeten Minerals. Der natürliche Schmelz wird durch die Schutzschicht vor Zahnbürstenabrasion geschützt.

Gegenstand der Erfindung ist eine Formulierung geeignet zur Abscheidung von Apatit, insbesondere ist sie geeignet zur biomimetischen Abscheidung von Apatit, ausgewählt aus Fluorapatit (Ca₅[F|(PO₄)₃), Hydroxylapatit (Ca₅[F|(PO₄)₃) oder deren Gemischen auf Zähnen oder auch auf einer Knochenmatrix von Wirbeltieren, wobei die Formulierung mindestens einen teilelastischen Formkörper, insbesondere dreidimensionalen, bevorzugt flächigen Formkörper, aufweist, umfassend mindestens eine Mineralisationsmatrix enthaltend mindestens ein Gel, und wobei der Formkörper zumindest teilweise in mindestens einer Ebene eine gegenüber wässrigen Medien verminderte Löslichkeit im Vergleich zur Mineralisationsmatrix aufweist, wobei die Ebene als Membran fungiert, und
a) die mindestens eine Mineralisationsmatrix ein Gel enthaltend wasserlösliche Phosphate oder zu wasserlöslichen Phosphat-Ionen hydrolysierbare Phosphate und einen pH-Wert von 2 bis 8 aufweist (Phosphat-Komponente), und
b) die mindestens eine oder eine zweite Mineralisationsmatrix ein zweites Gel umfassend Calcium-Ionen, insbesondere wasserlösliche Calcium-Ionen enthaltende Verbindungen, oder Calcium-Ionen freisetzende Verbindungen (Calcium-Komponente) mit einem pH-Wert von 3.5 bis 14 aufweist, umfassend Calcium-Ionen oder Calcium-Ionen freisetzende Verbindungen (Calcium-Komponente),
besonders bevorzugt liegt die mindestens eine Mineralisationsmatrix in einem ersten Formkörper I. und die zweite Mineralisationsmatrix in einem zweiten Formkörper II. vor, alternativ liegen zwei Mineralisationsmatrices in einem Formkörper vor.

Entsprechend einer bevorzugten Ausführungsform der Erfindung liegen das mindestens eine oder die zwei Mineralisationsmatrices jeweils unabhängig umfassend mindestens ein Gel in Form eines Flächenelements oder mindestens teilweisen Kiefernegativs vor. Ferner ist es bevorzugt, wenn der mindestens eine oder beide teilelastische Formkörper ebenfalls in Form eines Flächenelements oder mindestens teilweisen Kiefernegativs vorliegen.

Bevorzugt ist es, wenn die genannte mindestens eine Ebene eine an der äusseren Oberfläche angeordnete Ebene oder eine im Wesentlichen vollständige äussere Hülle ist.

Erfindungsgemässe Formulierungen umfassen ein oder zwei Formkörper in Form eines Flächenelements mit jeweils einer einschliessenden Hülle mit verminderter Löslichkeit, die insbesondere als Membran fungiert. In einer alternativen Ausführungsform umfasst die Formulierung einen Formkörper in Form eines Flächenelements mit jeweils einer einschliessenden Hülle mit verminderter Löslichkeit, die insbesondere als Membran fungiert, mit zwei aufeinander liegenden Mineralisationsmatrices jeweils umfassend ein Gel, wobei ein Gel die Phosphat- und ein Gel die Calcium-Komponenten umfasst. Diese beiden Mineralisationsmatrices werden von den Formkörpern A und B des Kits umfasst. Entsprechend einer weiteren alternativen Ausführungsform umfasst ein Flächenelement eine Mineralisationsmatrix mit einem Gel, wobei das Gel optional durch eine Membranschicht in zwei Reservoirs unterteilt ist umfassend einmal die Phosphat-und einmal die Calcium-Komponente. Die vorgenannten Formkörper liegen vorzugsweise als Flächenelement mit einer Schichtdicke von 5 bis 6000 µm vor, besonders bevorzugt sind Mineralisationsmatrices in Form eines Flächenelements mit jeweils einer Schichtdicke von 5 bis 3000 µm, bevorzugt 100 bis 600 µm, insbesondere 300 bis 600 µm oder um 500 µm plus/minus 200 µm, insbesondere plus/minus 100 µm, insbesondere. plus/minus 50 µm. Die erfindungsgemässen Formkörper umfassen zumindest teilweise eine Hülle oder mindestens eine Ebene mit verminderter Löslichkeit. Statt einem Flächenelement kann der Formkörper auch in Form eines zumindest teilweisen Kiefernegativs vorliegen, vorzugsweise als eine Negativform der Zähne des Ober- und/oder Unterkiefers. Flächenelement der Calcium-Komponente liegen vorzugswiese mit einer Schichtdicke von 500 bis 1500 µm vor, besonders bevorzugt sind Mineralisationsmatrices in Form eines Flächenelements mit jeweils einer Schichtdicke von 500 bis 1200 µm oder um 1000 µm plus/minus 100 µm, insbesondere plus/minus 50 µm. Flächenelement Phosphat-Komponente liegen vorzugsweise mit einer Schichtdicke von 100 bis 1000 µm vor, besonders bevorzugt sind Mineralisationsmatrices in Form eines Flächenelements mit jeweils einer Schichtdicke von 150 bis 800 µm, bevorzugt 300 bis 800 µm, insbesondere 400 bis 600 µm oder um 500 µm plus/minus 200 µm, insbesondere plus/minus 100 µm, insbesondere plus/minus 50 µm.

Erfindungsgemässe Formulierungen weisen nach der Vernetzung und wahlweise nach anschliessender Trocknung einen Wassergehalt von 8 bis 60 Gew.-%, bevorzugt von 30 bis 55 Gew.-% auf. Weiter bevorzugt weisen die Gele der Erfindungsgemässe Formulierungen nach der Vernetzung und wahlweise nach anschliessender Trocknung in der Mineralisationsmatrix der Phosphat-Komponente einen Wassergehalt von 20 bis 40 Gew.-%, bevorzugt von 25 bis 35 Gew.-%, besonders bevorzugt um 30 Gew.-% mit einer Abweichung von plus/minus 5 Gew.-% auf. Entsprechend bevorzugt sind Formulierungen der Calcium-Komponente, die nach der Vernetzung und wahlweise nach anschliessender Trocknung in der Mineralisationsmatrix einen Wassergehalt von 30 bis 60 Gew.-%, bevorzugt von 40 bis 60 Gew.-%, vorzugsweise um 50 Gew.-% mit einer Abweichung von plus/minus 5 Gew.-% aufweisen. Die so hergestellten Formulierungen können anschliessend luft-und feuchtigkeitsdicht in Blister, Sachets oder dergleichen eingeschweisst werden.

Die Formkörper können bevorzugt in folgenden Alternativen vorliegen: a) zwei Formkörper mit je einer teilweisen, vorzugsweise im Wesentlichen vollständigen Hülle, besonders bevorzugt mit einer umschliessenden Hülle, vorzugsweise zwei Formkörper mit einer Vernetzung der Ober- und/oder Unterseite der als Flächenelement vorliegenden Formkörper, b) ein Formkörper mit zwei Mineralisationsmatrices, der vorzugsweise durch eine Membran getrennt, c) ein Formkörper mit einer Mineralisationsmatrix umfassend zwei Gelbereiche, die optional durch eine Membran getrennt sind. Durch Verwendung von zwei Mineralisationsmatrices oder zwei Gelbereiche für die Phosphat-Komponente ist die Einstellung eines Konzentrationsprofils im Formkörper möglich. Entsprechend kann auch ein Konzentrationsprofil für einen Formkörper enthaltend die Calcium-Komponente eingestellt werden. Die Hülle oder die Ebene weist eine gegenüber wässrigen Medien verminderte Löslichkeit als die Mineralisationsmatrix auf, vorzugsweise fungiert die Hülle als Membran und vermindert ein Auflösen der Mineralisationsmatrix im Mundmilieu. Jedoch kann H₂O aus dem Speichel eindringen, und Fluoride, Calcium- und Phosphat-Ionen sowie Komposite oder Polypeptide können durch die Hülle diffundieren und auf der Zahnoberfläche als Apatit, Hydroxylapatit oder Fluorapatit abgeschieden werden. Die Hülle umschliesst eine wasserreiche Gelatinematrix aus der die Komposite, ionenbeladene Wassermoleküle bzw. hydratisierte Ionen heraus diffundieren können. Die Diffusion der Komposite ist wichtig, denn die Komposite sind mit organischen Makromolekülen substituierte Hydroxyapatite, die den Zahnschmelz bilden und, um diese auf den Zahnoberflächen abzuscheiden. Erfindungsgemäss werden Fluorapatit-Protein-Komposite aus den Formkörpern auf den Zahnoberflächen abgeschieden.

Erfindungsgemäss bilden die mindestens eine Ebene oder die Hülle die äussere Begrenzung mindestens einer Mineralisationsmatrix. Die Ebene oder Hülle kann auf unterschiedliche Weise erhalten werden, indem eine chemische Vernetzung der Mineralisationsmatrix durchgeführt wird oder durch Auftragen einer Beschichtung (coating) auf die Mineralisationsmatrix zur Ausbildung einer Ionen und Wasser durchlässigen Oberfläche der Mineralisationsmatrix, die als Membran fungiert. Die Vernetzung oder die Beschichtung können durch Tauchen, Auftragen, wie Pinseln, Sprühen, Rollen sowie durch weitere, dem Fachmann bekannte Massnahmen erfolgen. Die Ausbildung der Ebenen oder der Hülle kann auch mit unregelmässigen oder regelmässigen Durchbrechungen bzw. Poren oder unter Zugabe von Porenbildnern erfolgen.

Der aus der erfindungsgemässen Formulierung abgeschiedene Fluorapatit liegt vorzugsweise auf den Zähnen kristallin vor, bevorzugt microkristallin, besonders bevorzugt in Form von nadeligen Kristalliten. Die innerhalb eines Anwendungszyklus von etwa 8 Stunden auf den Zahnoberflächen abgeschiedene zumindest teilweise Apatitschicht, vorzugsweise durchgängige Apatitschicht, weist mindestens eine Schichtdicke von 1 µm, insbesondere 2 µm auf. Ebenso im Rahmen der Erfindung sind zumindest teilweise nicht durchgängige Apatitschichten, die unregelmässig bis vorzugsweise homogen zumindest einen Teil der behandelten Zahnoberfläche bedecken. Die Apatitschichten können zumindest teilweise bis zu 15 µm betragen, vorzugsweise werden im Wesentlichen vorzugsweise homogene Apatitschichten von größer gleich 2 µm, vorzugsweise größer gleich 5 µm bis 13 µm, und im Mittel von 2 bis 10 µm erhalten.

Unter einer gegenüber wässrigen Medien verminderten Löslichkeit der chemisch vernetzten Ebene oder Hülle im Vergleich zur Mineralisationsmatrix wird folgendes verstanden: Die chemisch unvernetzte Mineralisationsmatrix und optional nur mit einem Weichmacher modifizierte Mineralisationsmatrix, bspw. die mit Glycerin über Wasserstoffbrückenbindungen vorzugsweise als Addukt vernetzte Gelatine. Bevorzugt wird Gelatine der folgenden Qualitäten eingesetzt von Bloom 175 bis 300, oder höher, bevorzugt ist Gelatine Bloom 300 (Schweineschwarte).

Unter Zähnen von Wirbeltieren werden erfasst menschliche Zähne, Prothesen menschlicher Zähne, Milchzähne (*Dentes decidui*), Bleibende-Zähne (*Dentes permanentes*), Kronen, Inlays, Implantate, Zähne von Tieren, wie Haustieren und Nutztieren wie Hunden, Pferden, Katzen.

Unter einem mindestens teilelastischen Formkörper wird im Sinne der Erfindung ein dreidimensionaler Formkörper verstanden, der als Flächenelement oder auch mit jeder beliebigen dreidimensionalen Geometrie vorliegt, insbesondere in Form eines mindestens teilweisen Kiefernegativs, und elastische Eigenschaften aufweist. Als elastisch oder teilelastisch gilt der Formkörper, wenn der Körper unter Krafteinwirkung seine Form verändert und bei Wegfall der einwirkenden Kraft zumindest teilweise oder vollständig wieder in die Ursprungsform zurückkehrt. Die elastische oder teilelastische Eigenschaft weist der Formkörper vorzugsweise bei der Anwendung im Mundmilieu auf und vorzugsweise nach der Herstellung. Bei zu langer Trocknung kann die Elastizität abnehmen.

In einer nach der Erfindung besonders bevorzugten Formulierung weist die Mineralisationsmatrix in a) folgende Zusammensetzung auf: a) die mindestens eine Mineralisationsmatrix weist ein Gel auf, umfassend (i) wasserlösliche Phosphate oder zu wasserlöslichen Phosphat-Ionen hydrolysierbare Phosphate, insbesondere Na₂HPO₄, vorzugsweise liegt der Gehalt an Phosphat in der Mineralisationsmatrix bei 1 bis 10 Gew.-%, vorzugsweise von 2 bis 8 Gew.-%, besonders bevorzugt von 5 bis 8 Gew.-% (ii) einen Gehalt an Wasser oder ein Gemisch von Wasser und einem organischen Lösemittel, (iii) optional mindestens eine Carbonsäure, insbesondere eine Hydroxycarbonsäure, wie Milchsäure, und/oder ein Puffersystem, insbesondere liegt ein Puffersystem zur Einstellung des pH-Wertes im Bereich von 2 bis 8 vor, insbesondere von 3.5 bis 8, bevorzugt von 3.5 bis 6, besonders bevorzugt um 5,5 plus/minus 0,5. Der Gehalt bezieht sich auf PO₄³⁻.

Gleichzeitig weist die nach der Erfindung besonders bevorzugte Formulierung eine Mineralisationsmatrix in b) folgender Zusammensetzung auf: die zweite Mineralisationsmatrix oder die mindestens eine Mineralisationsmatrix weist ein zweites Gel auf, umfassend (i) Calcium-Ionen oder Calcium-Ionen freisetzende Verbindungen, insbesondere Calciumdichlorid oder Hydrate davon, vorzugsweise zusätzlich Calciumsulfat, Nanoapatit, Natriumcarbonat oder Calciumoxalat, vorzugsweise liegt der Gehalt an Calcium in der Mineralisationsmatrix bei 1 bis 10 Gew.-%, vorzugsweise größer gleich 1,5 bis 7,5 Gew.-%, (ii) optional Wasser oder ein Gemisch von Wasser und einem organischen Lösemittel, und (iii) optional mindestens eine Carbonsäure, wie eine Hydroxycarbonsäure bspw. Milchsäure, und/oder ein Puffersystem. Zur Herstellung der Puffer werden bevorzugt Fruchtsäuren und Alkali-Salze eingesetzt. Der Gehalt bezieht sich auf das Calcium (Ca²⁺).

Weiter ist es bevorzugt, wenn die Formulierung in der mindestens einen Mineralisationsmatrix ein Gel aufweist, umfassend mindestens ein wasserlösliches Fluorid (F⁻), mit Fluorid-Ionen, oder eine Fluoride freisetzende Verbindung. Besonders bevorzugt weist die Formulierung in a) als weitere Komponente (iv) mindestens ein wasserlösliches Fluorid oder eine Fluoride freisetzende Verbindung auf.

Nach einer bevorzugten Ausgestaltung der Erfindung umfasst das mindestens eine wasserlösliche Fluorid oder die mindestens eine Fluoride freisetzende Verbindung, insbesondere in a) die mindestens eine Mineralisationsmatrix, (i) mindestens eine unsubstituierte oder substituierte Alkyl-Gruppe aufweisende quaternäre mono- oder poly-Ammonium-Verbindung, vorzugsweise mit vier substituierten Alkyl-Gruppen, wobei die mindestens eine substituierte Alkyl-Gruppe umfasst Hydroxyalkyl-, Carboxyalkyl-, Aminoalkyl-Gruppen mit 1 bis 25 C-Atomen oder organofunktionelle durch Heteroatome unterbrochene Gruppen mit bis zu 50 C-Atomen. Bevorzugte Ammonium-Verbindungen können 1 bis 20 quaternäre Ammonium-Funktionalitäten enthalten, vorzugsweise 1, 2, 3, 4, 5, 6, 7, 8 Ammonium-Funktionalitäten, bevorzugt wird Olaflur (N,N,N'-Tris(2-hydroxyethyl)-N'-octadecyl-1,3-diaminopropandihydro-fluorid) als wasserlösliches Fluorid eingesetzt. Gleichfalls bevorzugt sind Aminfluoride, wie Oleaflur, Decaflur, Ethanolamin-Hydrofluorid, (ii) eine Fluoride freisetzende organofunktionelle Amino-Verbindung oder ein Fluoride freisetzendes Antiseptikum auf Basis von organofunktionellen Amino-Verbindungen, wie insbesondere Fluoride von *N*-Octyl-1-[10-(4-octyliminopyridin-1-yl)decyl]pyridin-4-imin, Cetylpyridinium fluorid, oder c) wasserlösliche anorganische Fluoride, wie Alkalifluoride, Natriumfluorid, Kaliumfluorid, Zinnfluorid, Ammoniumfluorid, oder Fluoride freisetzende anorganische Fluoride, wie Zinkfluorid, Zinkhydroxyfluorid.

Als erfindungsgemässes Gel kann vorzugsweise mindestens ein Gelbilder, ausgewählt aus denaturiertem Kollagen, Hydrokolloiden, Polypeptiden, Proteinhydrolysaten, synthetische Polyaminosäuren, Polysacchariden, Polyacrylaten (Superabsorber) oder Mischungen umfassend mindestens zwei der genannten Gelbilder in der Formulierung vorliegen. Insbesondere als zweite Mineralisationsmatrix und/oder zur Ausbildung der mindestens einen Ebene oder Hülle können vorzugsweise auch die Polysaccharide und/oder Polyacrylate zur Anwendung kommen. In der ersten Mineralisationsmatrix wird bevorzugt Gelatine eingesetzt, die erfindungsgemäss mit einem Weichmacher wie Glycerin oder einem anderen Polyol versetzt ist. Die Zugabe des Weichmachers verbessert die Handlingseigenschaften der Gelatine.

In einer erfindungsgemässen Formulierung umfasst die Mineralisationsmatrix als Gel Gelatine und vorzugsweise ein Weichmacher, bevorzugt ein Polyol, wie Glycerin und/oder deren Umsetzungsprodukte optional in Gegenwart von Wasser. Alternativ können auch Gelatine und ein Weichmacher wie Sorbitol eingesetzt werden. Der Weichmacher bewirkt eine Erhöhung des Schmelzbereiches durch Ausbildung von intermolekularen Wasserstoffbrückenbindungen. Als Gel wird erfindungsgemäss Gelatine vorzugsweise (denaturiertes Kollagen, tierisches Eiweiss, Protein), besonders bevorzugt wird eine sauer hydrolysiertes Kollagen, oder Gelatine und ein Polyol, wie Glycerin, eingesetzt. Alternativ können auch Casein, Stärkemehl, Cellulose, HPMC, Gummi Arabicum, Galactomannane, Guarkernmehl, Konjak, Xanthan, Calciumalginat, Dextran, Scleroglucan, Pektin, Carragenan (κ-, I- und λ-Carrageen), Agar-Agar, Alginat, Alginsäure, Natriumalginat, Calciumalginat, Traganth als Gel eingesetzt werden, wobei Gelatine oder Mischungen mit Gelatine bevorzugt sind.

Kern der Erfindung sind Formulierungen mit mindestens einem teilelastischen Formkörper, wobei der Formkörper mindestens einer Mineralisationsmatrix enthaltend mindestens ein Gel entspricht, wobei die Mineralisationsmatrix zumindest teilweise in mindestens einer chemisch mindestens teilvernetzten Ebene, insbesondere kovalent vernetzt, eine gegenüber wässrigen Medien verminderte Löslichkeit als die entsprechende nicht auf diese Art chemisch vernetzte Mineralisationsmatrix aufweist, in der sich lediglich intermolekulare Wasserstoffbrücken durch die Anwesenheit von Glycerin ausbilden, wobei die zumindest teilvernetzte Ebene, insbesondere als Membran fungiert, bevorzugt sind alle äusseren Oberflächen der Mineralisationsmatrix chemisch mindestens teilvernetzt. Für die Anwendung an Zähnen ist es bevorzugt, wenn die Mineralisationsmatrix und der Formkörper als ein Flächenelement oder mindestens teilweisen Kiefernegativs vorliegen. Ein erfindungsgemässes Flächenelement kann auch eine Oberflächentextur, die den Zahnoberflächen eines Zahnbogens nachempfunden ist, nachbilden. Entsprechend einer Ausführungsform der Erfindung umfasst die Formulierung mindestens eine Mineralisationsmatrix umfassend mindestens ein Gel in mindestens einem teilelastischen Formkörper, insbesondere flächigen Körper, wobei die Mineralisationsmatrix in Form eines Flächenelements vorliegt, und wobei der Formkörper mindestens zwei an der äusseren Oberfläche angeordneten Ebene oder eine äussere Hülle aufweist, die eine gegenüber wässrigen Medien verminderte Löslichkeit als die Mineralisationsmatrix aufweisen, und die insbesondere als Membran für chemische Verbindungen fungieren, wie die Komposite bspw. aus Polypeptiden und Salzen, sowie für Salze und Wasser. In dieser Ausführungsform ist es sinnvoll, dass der teilelastische Formkörper ebenfalls in Form eines Flächenelements vorliegt. In dieser Ausgestaltung ist die Formulierung besonders gut zur Behandlung mehrerer Zähne geeignet. Der Formkörper kann auch als mindestens teilweises Kiefernegativ vorliegen.

Verbindungen zur thermischen Stabilisierung des Gels umfassen vorzugsweise Weichmacher basierend auf Polyolen. Durch die erfindungsgemäss mögliche vollständige Umhüllung der Matrices kann je nach Anwendungsfall auf die Zugabe der Weichmacher verzichtet werden oder ein anderes, die Handlingseigenschaften der Gelatine verbesserndes Hilfsmittel zugesetzt werden. Bspw., wenn nur ein Zahn oder eine Kavität in einem Zahn mit einem Formkörper mit Hülle, vorzugsweise in Form eines Flächenelements oder mindestens teilweisen Kiefernegativs, Kugel, Granulatkorn oder Kapsel zur Apatitabscheidung versehen wird und innerhalb des Formkörpers die Bereiche der Gele optional durch eine Membran separiert vorliegen.

Als Verbindungen zur chemischen Vernetzung, insbesondere kovalenten Vernetzung, in der mindestens einen Ebene oder zur Ausbildung der Hülle der Mineralisationsmatrix werden die di- oder polyfunktionellen Vernetzer eingesetzt, bevorzugt Glutardialdehyd. Die chemischen Vernetzer bilden mit den Gelen, insbesondere den Polypeptiden oder Polyaminosäuren kovalente Vernetzungstellen aus. Vorzugsweise umfassen die di- oder polyfunktionellen Vernetzer Dialdehyde, Polyepoxide, und/oder Polyisocyanate sowie Mischungen umfassend mindestens zwei Vernetzer. Weiter bevorzugt werden pharmakologisch unbedenkliche Vernetzer eingesetzt. Bevorzugte Dialdehyde umfassen alpha, omega-Dialdehyde von Kohlenwasserstoffen, insbesondere umfassend 2 bis 50 C-Atome, insbesondere 4 bis 10 C-Atome in der bifunktionellen Alkylen Gruppe. Durch eine Behandlung der Mineralisationsmatrix mit einem Vernetzer verringert sich die Löslichkeit der Gelatine bis auf ein Niveau, dass sie für etwa 8 Stunden im Mundmilieu nicht verflüssigt. Bevorzugt ist die Behandlung mit Glutardialdehyd für mindestens 5 s, je nach Anwendung kann die Vernetzung länger erfolgen und damit stärker sein, bspw., wenn eine Mineralisationsmatrix für 12 bis 16 Stunden im Mundmilieu verbleiben soll. Nach 40 s Spülen in einer 0.5%igen Glutardialdehyllösung reduziert sich die Löslichkeit des Gels so weit, dass es bei Mundmillieu für bis zu 8 Stunden nicht verflüssigt. Die optional verwendbaren Membran(schichten) sind ionenfrei. Statt Spülen ist auch Tauchen oder Besprühen der Matrix mit der Lösung möglich.

Die Vernetzungslösung weist vorzugsweise einen Gehalt von etwa 0,25 bis 0,5 Gew.-% Vernetzer, vorzugsweise Glutardialdehyd auf. Die besten Ergebnisse hinsichtlich einer ausreichenden Vernetzung und optimalen Durchlässigkeit für die abzuscheidenden Apatitkomposite hat sich eine Behandlungszeit von, 0 bis 60 s, bevorzugt von etwa 5 bis 40 s, besonders bevorzugt von 10 bis 30 s, erfindungsgemäß um 20 Sekunden (s) gezeigt.

Entsprechend einer bevorzugten Ausführungsform umfasst die Formulierung (I) einen Formkörper in Form eines Flächenelements mit mindestens zwei optional durch eine Membran(schicht) getrennte Mineralisationsmatrices jeweils in Form eines Flächenelements enthaltend das Gel mit dem folgenden Schichtaufbau im Formkörper:
A) eine erste Mineralisationsmatrix in Form eines Flächenelements umfassend Gel und Phosphate oder zu wasserlöslichen Phosphat-Ionen hydrolysierbare Phosphate, wasserlösliche Fluoride oder Fluoride freisetzende Verbindung, Wasser oder ein Gemisch von Wasser und einem organischen Lösemittel, optional mindestens eine Carbonsäure und/oder ein Puffersystem,
B) optional Membran(schicht),
C) eine zweite Mineralisationsmatrix in Form eines Flächenelements umfassend Gel und Calcium-Ionen oder Calcium-Ionen freisetzende Verbindungen, optional Wasser oder ein Gemisch von Wasser und einem organischen Lösemittel, optional mindestens eine Carbonsäure und/oder ein Puffersystem, wobei (I) der Formkörper in Form eines Flächenelements mindestens eine oder zwei bis zu einem Vielflächner an der äusseren Oberfläche angeordnete Ebenen oder eine äussere Hülle aufweist, die (a) zumindest teilweise durch chemische Vernetzung erhältlich sind oder (b) einer Beschichtung entsprechen, und die insbesondere als Membran fungieren, und die eine gegenüber wässrigen Medien verminderte Löslichkeit als die Mineralisationsmatrices aufweisen, oder
erfindungsgemäss umfasst die Formulierung (II) zwei separate Formkörper jeweils unabhängig in Form eines Flächenelements mit mindestens jeweils einer optional durch eine Membran(schicht) getrennte, Mineralisationsmatrix in Form eines Flächenelements enthaltend das Gel, wobei der erste Formkörper umfasst eine erste Mineralisationsmatrix in Form eines Flächenelements umfassend Gel und Phosphate, wie Hydrogenphosphate, oder zu wasserlöslichen Phosphat-Ionen hydrolysierbare Phosphate, wasserlösliche Fluoride oder Fluoride freisetzende Verbindung, Wasser oder ein Gemisch von Wasser und einem organischen Lösemittel, optional mindestens eine Carbonsäure und/oder ein Puffersystem, optional Membran(schicht), und der
zweite Formkörper eine zweite Mineralisationsmatrix in Form eines Flächenelementes umfassend Gel und Calcium-Ionen oder Calcium-Ionen freisetzende Verbindungen, optional Wasser oder ein Gemisch von Wasser und einem organischen Lösemittel, optional mindestens eine Carbonsäure und/oder ein Puffersystem, aufweist,
wobei (II) die Formkörper jeweils mindestens eine oder zwei bis sechs an der äusseren Oberfläche angeordnete Ebenen oder eine äussere Hülle aufweisen, die (a) zumindest teilweise durch chemische Vernetzung erhältlich sind oder (b) einer Beschichtung entsprechen, und die insbesondere als Membran fungieren, und die eine gegenüber wässrigen Medien verminderte Löslichkeit als die Mineralisations-matrices aufweisen.

Gleichfalls Gegenstand der Erfindung sind Formulierungen umfassend (I) einen Formkörper in Form eines Flächenelements mit mindestens zwei optional durch eine Membran getrennte Mineralisationsmatrices jeweils unabhängig in Form eines Flächenelements enthaltend das Gel mit einer Schichtdicke von 50 bis 6000 µm, insbesondere von 500 bis 2000 µm, bevorzugt von 1000 bis 2000 µm oder 500 bis 1500 µm, oder (II) zwei separate Formkörper jeweils unabhängig in Form eines Flächenelements mit mindestens jeweils einer Mineralisationsmatrix in Form eines Flächenelements enthaltend das Gel, wobei jeder Formkörper jeweils unabhängig eine Schichtdicke von 10 bis 3000 µm aufweist, vorzugsweise 50 bis 1000 µm, besonders bevorzugt 100 bis 750 µm, vorzugsweise 300 bis 600 µm, besonders bevorzugt um 500 µm plus/minus 300 µm, wobei der erste Formkörper umfassend Phosphate, wie Hydrogenphosphate, oder zu wasserlöslichen Phosphat-Ionen hydrolysierbare Phosphate eine Schichtdicke von 50 bis 3000 µm, insbesondere 100 bis 3000 µm, bevorzugt von 300 bis 1000 µm, besonders bevorzugt um 500 µm plus/minus 200 µm oder +/- 50 µm, und/oder der zweite Formkörper umfassend Calcium-Ionen oder Calcium-Ionen freisetzende Verbindungen eine Schichtdicke von 10 bis 3000 µm aufweist, insbesondere 100 bis 3000 µm, bevorzugt von 300 bis 1000 µm, bevorzugt von 300 bis 750 µm, vorzugsweise um 500 µm plus/minus 200 µm oder +/- 50 µm. Alternativ sind die vorgenannten Formkörper ein Teil eines zumindest teilweisen Kiefernegativs mit einem entsprechenden inneren, an den Zähnen anzuordnenden mehrschichtigen Aufbau der Formkörper.

Die Carbonsäuren werden vorzugsweise ausgewählt aus Fruchtsäuren, wie α-Hydroxycarbonsäuren wie Äpfelsäure, Zitronensäure, Glycolsäure, Milchsäure und Weinsäure; Aminosäuren, Fettsäuren, Hydroxycarbonsäuren, Dicarbonsäuren und Mischungen umfassend mindestens zwei der genannten Säuren und/oder das Puffersystem umfasst Carboxylate von Alkylcarbonsäuren, Fettsäuren, Fruchtsäuren, Fumarate, Aminosäuren, Hydroxycarbonsäuren, Dicarbonsäuren und Mischungen umfassend mindestens zwei der genannten Säuren oder Phosphatpuffer. Für die Puffersysteme werden vorteilhaft Alkali- und/oder Erdalkali-Salze oder Zink-Salze eingesetzt.

Die Puffersysteme umfassen EDTA, TRIS: Tris(hydroxymethyl)-aminomethan für pH 7,2 bis 9,0, HEPES: 4-(2-Hydroxyethyl)-1-piperazinethanesulfonsäure für pH 6,8 bis 8,2, HEPPS: 4-(2-Hydroxyethyl)-piperazin-1-propansulfonsäure für pH 7,3 bis 8,7, Barbital-Acetat Puffer, MES: 2-(N-Morpholino)ethansulfonsäure für pH 5,2 bis 6,7, Kohlensäure-Bicarbonat-System für pH 6,2 bis 8,6; neutral, Kohlensäure-Silicat-Puffer für pH 5,0 bis 6,2; schwach sauer, Essigsäure-Acetat-Puffer für pH 3,7 bis 5,7, Phosphatpuffer: NaH₂PO₄ + Na₂HPO₄ für pH 5,4 bis 8,0, Ammoniakpuffer NH₃ + H₂O + NH₄Cl für pH 8,2 bis 10,2, Zitronensäure- oder Zitratpuffer. Besonders bevorzugte Puffersysteme umfassend Milchsäure Puffersysteme, EDTA, oder Barbital-Acetat Puffer und in der Mundspüllösung TRIS (Tris(hydroxymethyl)-aminomethan) Puffer. In der Mundspüllösung synonym zu Vorbehandlungslösung wird TRIS (Tris(hydroxymethyl)-aminomethan) eingesetzt.

Erfindungsgemäss einsetzbare Phosphate zur Herstellung der Phosphat enthaltenden Mineralisationsmatrices umfassen die Phosphate, Hydrogenphosphate, oder zu wasserlöslichen Phosphat-Ionen hydrolysierbare Phosphate umfassend a) Alkaliphosphate, Erdalkaliphosphate, Dihydrogenphosphate, Natriumdihydrogenphosphat, NaH₂PO₄, Kaliumdihydrogenphosphat, KH₂PO₄, Hydrogenphosphate, Dikaliumhydrogenphosphat, K₂HPO, Dinatriumhydrogenphosphat, Na₂HPO₄, Phosphatester, Monoester, Diester und Triester von Phosphaten, Natriumphosphat, Na₃PO₄, Kaliumphosphat, K₃PO₄, Calciumdihydrogenphosphat, Ca(H₂PO₄)₂, Monoester, Diester und Triester Calciumhydrogenphosphat, CaHPO₄, Calciumphosphat, Ca₃(PO₄)₂ und/oder
b) die Calcium-Ionen oder Calcium-Ionen freisetzende Verbindungen umfasst Calciumchlorid, Calciumdichlorid-Dihydrat, Calcium-Salz einer Carbonsäure umfassend Alkylcarbonsäuren, Hydroxycarbonsäure, Dicarbonsäuren, Fruchtsäuren, Aminosäuren, wie Calciumlactat, Calciumgluconat, Calciumlacto-Gluconat, Calciumalginat, Calcium-L-Ascorbat, in Wasser schlecht lösliche Calcium-Ionen retardiert freisetzende Verbindungen umfassend Calciumsulfat, Calciumapatit, Calciumcarbonat, Calciumoxalat, Calciumphosphat, Calciumalginat, vorzugsweise mit einer Partikelgrösse von kleiner 100 µm, bevorzugt um 10 µm, besonders bevorzugt kleiner gleich 5 µm bspw. bis 1 µm oder 50 nm oder vorzugsweise Gemische von wasserlöslichen und schlecht in Wasser löslichen Calcium-Ionen oder Calcium-Ionen freisetzenden Verbindungen. Die in Wasser schlecht löslichen Calcium-Ionen retardiert freisetzenden Verbindungen werden zur Texturverbesserung der teilweise klebrigen Gele dem gut wasserlösliche Calcium-Ionen enthaltendem Gel zugesetzt. In Bezug auf die Gesamtzusammensetzung der Mineralisationsmatrix umfassend das Gel können 1 bis 50 Gew.-% Wasser schlecht lösliche Calcium-freisetzende Verbindungen eingesetzt werden, bevorzugt werden 5 bis 30 Gew.-% eingesetzt.

Entsprechend einer Ausführungsform der Erfindung wird eine Formulierung zur Herstellung einer wässrigen Mundspüllösung zur Vorbehandlung der Zähne offenbart enthaltend mindestens ein gut wasserlösliches Calcium-Salz, umfassend vorzugsweise Calciumlactat, Calciumchlorid, Calciumgluconat, Calciumlacto-Gluconat, ein Hydrat der Salze oder eine Mischung enthaltend mindestens zwei der Salze, optional einen Gehalt eines Puffersystems, insbesondere TRIS (Tris(hydroxymethyl)-aminomethan), optional einen Gehalt an Maskierungsmittel oder Aromatisierungsmittel sowie üblichen Formulierungshilfsmitteln zur Herstellung einer Vorbehandlungslösung oder zusammen mit Wasser als Vorbehandlungslösung vor einer Behandlung in der Apatit auf Zähnen von Wirbeltieren abgeschieden wird. In Mundspüllösung (Vorbehandlungslösung) wird TRIS (Tris(hydroxymethyl)-aminomethan) eingesetzt. Gegenstand der Erfindung ist auch eine Formulierung in Form einer wässrigen Mundspüllösung umfassend Wasser, 0,01 bis 2 mol eines gut wasserlöslichen Calcium-Salzes in Bezug auf die Gesamtzusammensetzung, optional 0,01 bis 0,5 mol, vorzugsweise zu 0,05 bis 0,2 mol eines Puffersystems, insbesondere TRIS (Tris(hydroxymethyl)-aminomethan), optional Maskierungsmittel oder Aromatisierungsmittel und aufweisend einen pH-Wert von 5.0 bis 12.0. Die Mundspüllösung bzw. Vorbehandlungslösung kann auch zur Behandlung einzelner Zähne eingesetzt werden.

Nach einer weiteren Alternative wird eine Formulierung einer wässrigen Mundspüllösung offenbart, vorzugsweise zur Verwendung mit einer vorgenannten erfindungsgemässen Formulierung, umfassend Wasser, 0.1 bis 30 Gew.-% eines gut wasserlöslichen Calcium-Salzes, insbesondere 5 bis 20 Gew.-%, bevorzugt 5 bis 15 Gew.-% in Bezug auf die Gesamtzusammensetzung, vorzugsweise umfassend Calciumlactat, Calciumchlorid, Calciumgluconat, Calciumlacto-Gluconat, ein Hydrat der Salze oder eine Mischung enthaltend mindestens zwei der Salze, optional mit einem Gehalt eines Puffersystems, insbesondere Tris, optional Maskierungsmittel oder Aromatisierungsmittel und aufweisend einen pH-Wert von 5.0 bis 12.0.

Gleichfalls Gegenstand der Erfindung ist ein Verfahren zur Herstellung einer Phosphat-Ionen enthaltenden Formulierung sowie eine Formulierung erhältlich nach dem Verfahren zur Abscheidung von Apatit, insbesondere zur biomimetischen Abscheidung von Apatit, ausgewählt aus Fluorapatit, Hydroxylapatit oder deren Gemischen auf Zähnen von Wirbeltieren, indem,
(1) mindestens ein teilelastischer Formkörper, insbesondere Formkörper A, umfassend mindestens eine Mineralisationsmatrix enthaltend mindestens ein Gel mit wasserlöslichen Phosphaten oder zu wasserlöslichen Phosphat-Ionen hydrolysierbare Phosphate hergestellt wird, wobei der Formkörper zumindest teilweise in mindestens einer Ebene, eine gegenüber wässrigen Medien verminderte Löslichkeit als die Mineralisationsmatrix aufweist, insbesondere an mindestens einer äusseren Oberfläche, besonders bevorzugt mehrere Ebenen, die eine Hülle bilden, wobei die Ebene insbesondere als H₂O, Peptid und Ionen-gängige Membran fungiert, und der Formkörper hergestellt wird, indem
a) zur Herstellung mindestens einer Mineralisationsmatrix enthaltend das Gel, auch Phosphatkomponente A genannt, in einem ersten Schritt eine Mischung von
(i) 0.05 bis 4 mol/l, 0.5 bis 1.5 mol/l wasserlöslichen Phosphaten oder zu wasserlöslichen Phosphat-Ionen hydrolysierbaren Phosphaten,
(ii) eine entsprechende Menge Wasser oder ein Gemisch von Wasser und einem organischen Lösemittel,
(iii) optional mindestens eine Carbonsäure und/oder ein Puffersystem, insbesondere zur Einstellung des pH-Wertes von 2 bis 8, vorzugsweise 3.5 bis 8, bevorzugt 3.5 bis 6, besonders bevorzugt um 5,5 plus/minus 0,5
(iv) 0 bis 6000 Gew.-ppm wasserlösliches Fluorid oder eine Fluoride freisetzende Verbindung, insbesondere 1 bis 4000 Gew.-ppm, weiter bevorzugt 500 bis 2500 Gew.-ppm, besonders bevorzugt um 2000 Gew.-ppm plus/minus 500 Gew.-ppm, gemischt werden, in einem weiteren Schritt wird aus der in a) hergestellten Mischung
b) mit Gelatine und optional Glycerin unter Erwärmen das Gel hergestellt,
c) Formung unter Ausbildung der Mineralisationsmatrix, optional Verfestigung, und in einem nachfolgenden Schritt d) erfolgt eine Ausbildung einer an der äusseren Oberfläche angeordneten Ebene, insbesondere der Hülle, der mindestens einen Mineralisationsmatrix unter Bildung des Formkörpers.

Die Ebene oder Hülle kann durch Vernetzung oder Auftragen eines Coatings (Beschichtung) erzeugt werden. Zur Vernetzung der mindestens einen an der äusseren Oberfläche angeordneten Ebene der mindestens einen Mineralisationsmatrix, insbesondere in Form eines Flächenelements oder mindestens teilweisen Kiefernegativs, wird diese in einem weiteren Schritt mit einer Mischung, vorzugsweise einer wässrigen Lösung, enthaltend einen di- oder polyfunktionellen Vernetzer in Kontakt gebracht.

Die Phosphat-Lösung zur Herstellung der Phosphatkomponente A enthält unter anderem ein wasserlösliches Phosphat-Salz. Geeignet sind z.B. Alkalisalze wie Natrium-oder Kaliumphosphate, Hydrogen- oder Dihydrogenposphate. Die Aufzählung ist einschliesslich aber nicht ausschliesslich. Die Konzentration der Phosphatsalze in der Lösung beträgt zwischen 0.05 bis 4 mol/l Gel, bevorzugt 0.5 bis 1.5 mol/l, besonders bevorzugt um 1 mol/l plus/minus 0,5 mol/l. Die Phosphatlösung enthält ausserdem ein wasserlösliches Fluoridsalz z.B. ein Alkalisalz, oder Zinnfluorid oder Olafluor. Die Aufzählung ist einschliesslich aber nicht ausschliesslich. Die Konzentration des Fluorids liegt in der Lösung zwischen 0 bis 6000 Gew.-ppm, bevorzugt 200 bis 4000 Gew.-ppm, besonders bevorzugt 2500 bis 4000 Gew.-ppm oder um 3000 Gew.-ppm plus/minus 500 Gew.-ppm. Die Phosphat-Lösung kann durch Zugabe des Vernetzers, beispielsweise Glutardialdehyd, als Vernetzerlösung verwendet werden.

Der pH-Wert der Phosphat-Lösung liegt zwischen 2.0 und 8.0, bevorzugt zwischen 3.5 und 5.5 wird mit einem geeigneten Puffersystem eingestellt. Besonders geeignet sind Carbonsäuren wie Ascorbinsäure. Brenztraubensäure, Weinsäure, Essigsäure, Milchsäure oder Äpfelsäure aber auch alle anderen Puffersysteme. Die Konzentration des Puffers liegt zwischen 0.25 und 4.0 mol/l, bevorzugt zwischen 0.5 und 1.5 mol/l.

Aus der Lösung wird ein Gelatine-Glycerin-Gel hergestellt. Die Menge an Gelatine beträgt vorzugsweise 25 bis 40 Gew.-% und die Menge an Glycerin 5 bis 20 Gew.-% in Bezug auf die Gesamtzusammensetzung an wässrigem Gel. Um die Komponenten homogen zu mischen, wird die Zubereitung auf 40 bis 90°C erwärmt bevorzugt auf 50 bis 70°C. Die Dicke der Phosphat-Komponente A beträgt hierbei 50 bis 3000 µm, bevorzugt 200 bis 2000 µm, besonders bevorzugt 300 bis 1500 µm.

Ebenso Gegenstand der Erfindung ist ein Verfahren zur Herstellung einer Calcium-Ionen enthaltenden Formulierung sowie eine Formulierung erhältlich nach dem Verfahren zur Abscheidung von Apatit, insbesondere zur biomimetischen Abscheidung von Apatit, ausgewählt aus Fluorapatit Hydroxylapatit oder deren Gemischen auf Zähnen von Wirbeltieren indem,
(1) mindestens ein teilelastischer Formkörper, insbesondere Formkörper B, umfassend mindestens eine Mineralisationsmatrix enthaltend mindestens ein Gel mit Calcium-Ionen oder Calcium-Ionen freisetzende Verbindungen hergestellt wird, wobei der Formkörper zumindest teilweise in mindestens einer Ebene, eine gegenüber wässrigen Medien verminderte Löslichkeit als die Mineralisationsmatrix aufweist, insbesondere an mindestens einer äusseren Oberfläche, besonders bevorzugt mehrere Ebenen, die eine Hülle bilden, wobei insbesondere die Ebene als Membran fungiert, und der Formkörper hergestellt wird, indem
a) zur Herstellung mindestens einer Mineralisationsmatrix enthaltend das Gel, auch Calciumkomponente B genannt, in einem ersten Schritt eine Mischung von
(i) 0.1 bis 2 mol/l Calcium-Ionen oder Calcium-Ionen freisetzende Verbindungen,
(ii) eine entsprechende Menge Wasser oder ein Gemisch von Wasser und einem organischen Lösemittel,
(iii) optional mindestens eine Carbonsäure und/oder ein Puffersystem, insbesondere zur Einstellung des pH-Wertes von 3.5 bis 14, bevorzugt von 4.0 bis 6.0 oder 6.0 bis 11.0, vorzugsweise 4.0, besonders bevorzugt um 4.0 plus/minus 0,5,
gemischt werden, in einem weiteren Schritt wird aus der in a) hergestellten Mischung
b) mit Gelatine und optional Glycerin unter Erwärmen das Gel hergestellt,
c) Formung unter Ausbildung der Mineralisationsmatrix, optional Verfestigung, und in einem nachfolgenden Schritt d) erfolgt eine Ausbildung einer an der äusseren Oberfläche angeordneten Ebene, insbesondere der Hülle, der mindestens einen Mineralisationsmatrix unter Bildung des Formkörpers. Die Ebene oder Hülle kann durch die vorgenannte Vernetzung oder Auftragen eines Coatings erzeugt werden. Entscheidend bei der Herstellung der Ebene oder der Hülle ist eine gewisse Porosität, um eine Abscheidung der Biokomposite zu ermöglichen.

Zur Herstellung der vorgenannten Formulierungen werden in b) jeweils unabhängig im weiteren Schritt 5 bis 50 Gew.-% Gelatine in Bezug auf die Gesamtzusammensetzung Gel zugesetzt und 0 bis 30 Gew.-% Glycerin in Bezug auf die Gesamtzusammensetzung Gel zugesetzt, bevorzugt werden 25 bis 40 Gew.-% Gelatine und 5 bis 20 Gew.-% Glycerin zur Herstellung der Formulierung enthaltend wasserlösliche Phosphate oder zu wasserlöslichen Phosphat-Ionen hydrolysierbaren Phosphate enthaltenden Matrix, und 20 bis 40 Gew.-% Gelatine und 15 bis 25 Gew.-% Glycerin zur Herstellung der Formulierung enthaltend Calcium-Ionen oder Calcium-Ionen freisetzende Verbindungen enthaltenden Mineralisationsmatrix zugesetzt.

Die Gelatine enthaltenden Formulierungen in Schritt b) werden vorzugsweise auf 40 bis 90 °C erwärmt, um die Komponenten homogen zu vermischen, bevorzugt ist der Temperaturbereich von 50 bis 70 °C.

Die Lösung zur Herstellung der Calciumkomponente B enthält ein wasserlösliches Calciumsalz, z.B. Calciumchlorid oder Calciumlactat oder Calciumgluconat oder Calciumlacto-Gluconat. Die Aufzählung ist einschliesslich aber nicht ausschliesslich. Die Konzentration liegt zwischen 0.1 und 2.0 mol/l, bevorzugt zwischen 0.5 und 1.5 mol/l. Der pH-Wert zwischen 4.0 und 14.0, bevorzugt zwischen 6.0 und 11.0 wird mit einem geeigneten Puffersystem eingestellt. Besonders geeignet sind Carbonsäuren wie Ascorbinsäure, Brenztraubensäure, Weinsäure, Essigsäure, Milchsäure oder Äpfelsäure aber auch alle anderen Puffersysteme mit geeignetem pks-Wert. Die Konzentration des Puffers liegt zwischen 0.1 und 3.0 mol/l, bevorzugt zwischen 0.25 und 1.0 mol/l. Aus der Lösung wird ein Gelatine-Glycerin-Gel hergestellt. Die Menge an Gelatine beträgt vorzugsweise 20 bis 40 Gew-% in Bezug auf die Gesamtzusammensetzung an wässrigem Gel und die Menge an Glycerin 15 bis 25 Gew-%. Da die Calcium-Gelatine-Lösung auch nach Gelieren sehr klebrig ist und damit unangenehm im Handling wird zur Texturverbesserung ein schlecht lösliches Calciumsalz zugegeben. Besonders geeignet sind Calciumsulfat, Calciumapatit, Calciumcarbonat, Calciumoxalat. Die Aufzählung ist einschliesslich aber nicht ausschliesslich. Um eine besonders homogene Paste zu erhalten, ist es vorteilhaft wenn die Partikelgrössen unter 10 µm liegen. Bevorzugt werden Partikel mit Partikelgrössen kleiner 1 µm eingesetzt. Die Zugabe des schwer löslichen Calciumsalzes liegt bevorzugt bei 1 bis 50 %, sehr bevorzugt bei 5 bis 30%. Um die Komponenten homogen zu mischen, wird die Zubereitung unter Rühren auf 40-90°C erwärmt bevorzugt auf 50 bis 70°C.

Die Dicke der Calcium-Komponente A beträgt hierbei 10 bis 3000 µm, bevorzugt 100 bis 1500 µm, besonders bevorzugt 300 bis 1500 µm, vorzugsweise 500 bis 1500 µm.

Zur Herstellung der Formkörper der Formulierungen werden die noch nicht verfestigten Gele geformt und anschliessend verfestigt. Daher ist ebenfalls Gegenstand der Erfindung ein Verfahren, indem das im weiteren Schritt b) hergestellte Gel geformt wird, das verfestigbar ist. Bevorzugt sind Flächenelemente oder eine individuelle dreidimensionale Form, vorzugsweise in Form des Kiefernegativs.

Die Formung der Gele zur Ausbildung der Mineralisationsmatrixe kann durch Einfüllen in Formen, Extrusion, Formung zu Flächenelementen durch Aufstreichen, Verteilen, Ausrollen, Pressen durch entsprechend geformte Düsen erfolgen, gefolgt von einem Verfestigungsschritt. Eine Extrusion der Gele in jede beliebige Form ist vorzugsweise mit pastenförmigen Gelen gut möglich. Die Verfestigung erfolgt in der Regel bereits beim Abkühlen. Generell kann das Gel in jede beliebige Form überführt und verfestigt werden und optional mit einer im Wesentlichen vollständigen oder teilweisen Hülle versehen werden, um ein Auflösen im Mundmilieu oder unter physiologischen Bedingungen zu vermeiden.

Um das Auflösen der Mineralisationsmatrices im Mundmilieu zu verhindern, werden die Mineralisationsmatrices chemisch vernetzt. Dabei ist der Grad der Vernetzung so zu wählen, dass die Polypeptide immer noch für die Komposit-Bildung zur Verfügung stehen, aber dennoch eine ausreichende Festigkeit im Mund-Milieu bzw. unter physiologischen Bedingungen gewährleistet ist. Das Gel befindet sich bei der Vernetzung sinnvollerweise bereits in der gewünschten Form. Das können entweder Streifen bestimmter Dimension sein oder dreidimensionale Körper in Form des Kiefernegativs. Nach der Vernetzung und wahlweise anschliessender Trocknung auf einen Wassergehalt zwischen 8 bis 60 Gew.-%, bevorzugt 30 bis 55 Gew.-% können die Gelformen unter Erhalt der zwei- oder dreidimensionalen Form entnommen werden.

Die Trocknung stabilisiert die Gelformen zusätzlich. Der Grad der Vernetzung kann über die Art und Konzentration des Vernetzungsmittels, dem pH-Wert der Vernetzungslösung und die Einwirkzeit eingestellt werden. Zur Vernetzung sind alle di- oder polyfunktionellen Verbindungen geeignet, die mit mehreren Seitengruppen der Gelatine reagieren können, wie Dialdehyde, Polyepoxide oder Poliisocyanate. Bevorzugt verwendet wird Glutardialdehyd, welches ferner den Vorteil der desinfizierenden Wirkung hat und leicht flüchtig ist.

Die Vernetzung mindestens einer an der äusseren Oberfläche angeordneten Ebene der mindestens einen Mineralisationsmatrix vorzugsweise in Form eines Flächenelements oder mindestens teilweisen Kiefernegativs, erfolgt in einem weiteren Schritt mit einer Mischung, vorzugsweise einer wässrigen Lösung, enthaltend einen di- oder polyfunktionelle Vernetzer umfassend Dialdehyde, Polyepoxide, Polyisocyanate durch in Kontaktbringen mit der Mineralisationsmatrix. In diesem Schritt bildet sich die im Wesentlichen wasserunlösliche Vernetzung mit dem Gel aus. Durch das in Kontaktbringen erfolgt eine zumindest teilweise chemische Vernetzung in mindestens einer an der äusseren Oberfläche angeordneten Ebene des Flächenelements, unter Erhalt des mindestens einen Formkörpers umfassend mindestens eine Ebene mit einer gegenüber wässrigen Medien verminderten Löslichkeit im Vergleich zur Mineralisationsmatrix. Entsprechend einer Alternative werden alle an den äusseren Oberflächen angeordneten Ebenen des Formkörpers, wie Flächenelementes vernetzt, um eine vernetzte Hülle der mindestens einen Mineralisationsmatrix herzustellen unter Erhalt des mindestens einen Formkörpers.

Gegenstand der Erfindung sind auch zwei Vernetzerlösungen, insbesondere zur Verwendung bei der Herstellung jeweils unabhängig einer Formulierung in Form eines Formkörpers, mit einer Vernetzerlösung (a) umfassend eine Phosphat-Mischung, die hergestellt wird durch Mischen von
(i) 0.05 bis 4 mol/l, 0.5 bis 1.5 mol/l wasserlöslichen Phosphaten oder zu wasserlöslichen Phosphat-Ionen hydrolysierbaren Phosphaten,
(ii) einer entsprechenden Menge Wasser oder ein Gemisch von Wasser und einem organischen Lösemittel,
(iii) optional mindestens einer Carbonsäure und/oder eines Puffersystems
(iv) 0 bis 6000 Gew.-ppm wasserlöslichen Fluorids oder einer Fluorid freisetzenden Verbindung, und/oder einer Vernetzerlösung (b) umfassend eine Calcium-Mischung, die hergestellt wird durch Mischen von
(i) 0.1 bis 2 mol/l Calcium-Ionen oder Calcium-Ionen freisetzenden Verbindungen,
(ii)einer entsprechenden Menge Wasser oder einem Gemisch von Wasser und einem organischen Lösemittel,
(iii) optional mindestens einer Carbonsäure und/oder eines Puffersystems gemischt werden, und (a) und/oder (b) mit einer definierten Menge einer Lösung enthaltend einen di- oder polyfunktionellen Vernetzer umfassend Dialdehyde, Polyepoxide, Polyisocyanate, gemischt werden, insbesondere ist der Vernetzer Glutardialdehyd. Die Phosphat-Lösung kann durch Zugabe des Vernetzers, beispielsweise Glutardialdehyd, als Vernetzerlösung verwendet werden. Ebenso kann die CalciumLösung durch Zugabe des Vernetzers, beispielsweise Glutardialdehyd, als Vernetzerlösung verwendet werden.

Der Vernetzung liegt dazu in einer Vernetzerlösung vor und wird mit dem geformten Gel in Kontakt gebracht. Bevorzugt sind Lösungen von 0,005 bis 90 Gew.-% Vernetzer in Lösemittel in Bezug auf die Gesamtzusammensetzung, insbesondere in Wasser oder Wasser enthaltendem Lösemittel, bevorzugt sind 0,005 bis 5 Gew-%, besonders bevorzugt 0,1 bis 4 Gew.-%, vorteilhaft um 0,1 bis 1 Gew.-%. Bevorzugt wird als Vernetzerlösung eine wässrige Glutardialdehyd-Lösung eingesetzt. Die Vernetzerlösung wird vorzugsweise hergestellt, indem die Phosphatlösung mit dem Vernetzer versetzt wird. Die bevorzugte Einwirkzeit liegt bei 1 bis 200 Sekunden, besonders bevorzugt bei 10 bis 60 Sekunden (s). Vorzugsweise wird für etwa 20 Sekunden behandelt. Der bevorzugte pH-Wert der Vernetzungslösung liegt zwischen 4.0 und 12.0. Ein anschliessendes Abspülen mit Phosphat- bzw. Calciumlösung ist möglich. Gegenstand der Erfindung ist auch eine Vernetzerlösung umfassend eine Phosphatlösung oder eine Calciumlösung sowie einen Gehalt an Vernetzer.

Zur Herstellung von Formkörpern enthaltend jeweils eine Matrix wird wie folgt verfahren: mindestens eine Mineralisationsmatrix, insbesondere in Form eines Flächenelementes oder mindestens teilweisen Kiefernegativs, zur Herstellung eines Phosphate und Fluoride enthaltenden Formkörpers wird in mindestens einer an der äusseren Oberfläche angeordneten Ebene oder als Hülle vernetzt. Entsprechend wird mit einer Mineralisationsmatrix zur Herstellung eines Calcium-Ionen enthaltenden Formkörpers verfahren. Die Herstellung von Formkörpern enthaltend beide Mineralisationsmatrices kann wie folgt erfolgen: Anordnung (i) mindestens eine Mineralisationsmatrix enthaltend ein Gel in Form eines Flächenelementes umfassend Phosphate und Fluoride, (ii) optional getrennt durch eine Membran-(schicht) und (iii) eine darauf angeordnete zweite Mineralisationsmatrix enthaltend ein Gel in Form eines Flächenelementes umfassend Calcium-Ionen. Äussere Vernetzung oder Beschichtung des die Flächenelemente (i), (iii) und optional (ii) enthaltenden Formkörpers zur Ausbildung der Hülle. Alternativ kann eine Beschichtung einer oder beider Mineralisationsmatrices umfassend eine Beschichtung mit membranbildenen polymeren Überzügen, insbesondere mit Acrylaten wie EUDRAGIT-Polymeren oder Polyvinylalkohol (PVA) bspw. von Merck Millipore, erfolgen.

Ferner ist ein Gegenstand der Erfindung ein Kit aufweisend einen teilelastischen Formkörper A, insbesondere eine Formulierung eines teilelastischen Formkörpers A, und einen teilelastischen Formkörper B, insbesondere eine Formulierung eines teilelastischen Formkörpers B, die jeweils unabhängig in Form eines dreidimensionalen Körpers, insbesondere als Flächenelement oder mindestens teilweisen Kiefernegativ, vorliegen, wobei (a) der teilelastische Formkörper A umfasst, (a1) mindestens eine Mineralisationsmatrix umfassend mindestens ein Gel, (a2) mindestens ein wasserlösliches Phosphat oder zu wasserlöslichen Phosphat-Ionen hydrolysierbare Phosphate, und (a3) optional mindestens ein Carbonsäure und/oder ein Puffersystem, (a4) optional wasserlösliche Fluoride oder eine Fluoride freisetzende Verbindung, (a5) optional einen Gehalt an Wasser oder ein Gemisch von Wasser und einem organischen Lösemittel,
(b) der teilelastische Formkörper B umfasst, (b1) mindestens eine Mineralisations-matrix umfassend mindestens ein Gel, (b2) wasserlösliche Calcium-Ionen oder Calcium-Ionen freisetzende Verbindungen, und
(b3) optional mindestens eine Carbonsäure und/oder ein Puffersystem (b4) optional Wasser oder ein Gemisch von Wasser und einem organischen Lösemittel, oder mindestens einen teilelastischen Formkörper C, wobei der (c) teilelastische Formkörper C umfasst, (c1) mindestens eine Mineralisationsmatrix umfassend mindestens ein Gel, (c1.1) mindestens ein wasserlösliches Phosphat oder zu wasserlöslichen Phosphat-Ionen hydrolysierbares Phosphat, und (c1.2) optional mindestens ein Carbonsäure und/oder ein Puffersystem, (c1.3) optional wasserlösliche Fluoride oder eine Fluoride freisetzende Verbindung, (c1.4) optional einen Gehalt an Wasser oder ein Gemisch von Wasser und einem organischen Lösemittel, (c2) optional eine Membran(schicht), (c3) mindestens einer Mineralisationsmatrix umfassend mindestens ein Gel, (c3.1) wasserlösliche Calcium-Ionen oder Calcium-Ionen freisetzende Verbindungen, und (c3.2) optional mindestens eine Carbonsäure und/oder ein Puffersystem, (c3.3) optional Wasser oder ein Gemisch von Wasser und einem organischen Lösemittel, wobei der teilelastische Formkörper C den Schichtaufbau c1 und c3 oder c1, c2 und c3 aufweist. Der Formkörper C kann alternativ auch zwei Mineralisationsmatrices c1 und c1' mit unterschiedlichen Gehalten an Phosphat aufweisen, um einen Konzentrationsgradienten in der Phosphat-Komponente vorzusehen. Entsprechend kann ein Formkörper C auch zwei Mineralisationsmatrices c3 und c3' unterschiedlicher Konzentrationen aufweisen.

Der Wassergehalt der Mineralisationsmatrices ändert sich im Wesentlichen nicht. Wobei auch die Mineralisationsmatrices vorzugsweise jeweils unabhängig in Form eines Flächenelements oder mindestens teilweisen Kiefernegativs vorliegen.

Sowie ferner ein Kit in dem der jeweilige Formkörper jeweils unabhängig zumindest teilweise in mindestens einer an der äusseren Oberfläche angeordneten Ebene oder eine äussere Hülle aufweist, wobei die Ebene oder Hülle eine gegenüber wässrigen Medien verminderte Löslichkeit im Vergleich zur Mineralisationsmatrix aufweist, wobei die Ebene oder Hülle als Membran fungiert. Sowie ein Kit umfassend (I) einen Formkörper in Form eines Flächenelements mit mindestens zwei optional durch eine Membran getrennte Mineralisationsmatrices jeweils unabhängig in Form eines Flächenelements enthaltend das Gel mit einer Schichtdicke von 50 bis 6000 µm, oder (II) zwei separate Formkörper jeweils unabhängig in Form eines Flächenelements mit mindestens jeweils einer Mineralisationsmatrix in Form eines Flächenelements enthaltend das Gel, wobei jeder Formkörper jeweils unabhängig eine Schichtdicke von 10 bis 3000 µm aufweist, wobei der erste Formkörper umfassend Phosphate oder zu wasserlöslichen Phosphat-Ionen hydrolysierbare Phosphate eine Schichtdicke von 50 bis 3000 µm und der zweite Formkörper umfassend Calcium-Ionen oder Calcium-Ionen freisetzende Verbindungen eine Schichtdicke von 10 bis 3000 µm aufweist. In der Alternative des Kiefernegativs umfasst der Formkörper vorzugsweise innere an den Zähnen anliegende Schichten der mindestens einen oder zwei Mineralisationsmatrices. Die Schichten können beispielsweise durch Tauchen, Aufpinseln oder andere dem Fachmann bekannte Massnahmen angebracht werden und nachfolgend vernetzt werden. Das Kiefernegativ kann daher einen Träger sowie die Mineralisationsmatrices als auch direkt aus den Mineralisationsmatices hergestellt sein, bspw. bildet die Calcium-Komponente den Träger und die Phosphat-Komponente wird als innere Schicht in Form eines nicht ebenen Flächenelementes eingebracht.

Ferner umfasst das Kit vorzugsweise eine Formulierung in Form a) einer wässrigen Vorbehandlungslösung synonym zu Mundspüllösung umfassend Wasser, 0.1 bis 30 Gew.-% eines gut wasserlöslichen Calcium-Salzes, insbesondere 5 bis 15 Gew.-% in Bezug auf die Gesamtzusammensetzung, vorzugsweise Calciumlactat, Calciumchlorid, Calciumgluconat, Calciumlacto-Gluconat, ein Hydrat der Salze oder eine Mischung enthaltend zwei der Salze, optional einen Gehalt eines Puffersystems, optional Maskierungsmittel oder Aromatisierungsmittel und aufweisend einen pH-Wert von 5.0 bis 12.0, oder die Formulierung in Form b) umfasst mindestens ein gut wasserlösliches Calcium-Salz, umfassend vorzugsweise Calciumlactat, Calciumchlorid, Calciumgluconat, Calciumlacto-Gluconat, ein Hydrat der Salze oder eine Mischung enthaltend zwei der Salze, optional einen Gehalt eines Puffersystems, optional einen Gehalt an Maskierungsmittel oder Aromatisierungsmittel sowie üblichen Formulierungshilfsmitteln, wie Sprengmittel, HPMC etc., insbesondere in Form eines wasserlöslichen Granulats, wasserlöslichen Pellets, Tabletten, als Sachet, Pulver bzw. Granulat in einem Sachet oder löslichen Kapseln. Gegenstand der Erfindung ist auch die Verwendung einer einzelnen Formulierung der Form b) zusammen mit einem Kit umfassend die erfindungsgemässen Formulierungen. Die vorgenannte Mundspüllösung ist eine Vorbehandlungslösung zur Vorbehandlung einzelner oder aller Zähne oder eine Formulierung aus der durch Zugabe von Wasser eine entsprechende Lösung hergestellt werden kann.

Die Zusammensetzung des Kits ist im Folgenden beschrieben. Die Mundspülung oder Vorbehandlungslösung ist zusammengesetzt aus 0.1 Gew.-% bis 30 Gew.-% eines gut wasserlöslichen Calcium-Salzes, bevorzugt 5 Gew.-% bis 15 Gew.-% Calciumchlorid oder Calciumlactat oder Calciumgluconat oder Calciumlacto-Gluconat oder anderer ausreichend lösliche Calciumsalze. Die Lösung wird mit einem geeigneten Puffer auf einen pH-Wert zwischen 5.0 und 12.0 eingestellt, bevorzugt 8,0 bis 10,0. Zur Geschmacksverbesserung ist eine Aromatisierung oder auch eine Komplexierung von geschmacksbeeinträchtigenden Verbindungen möglich, solange die Apatitabscheidung nicht beeinträchtig wird. Als Puffer eignen sich alle Puffer die in diesem pH-Wert-Bereich gute Pufferkapazität aufweisen z.B. EDTA, Tris, HEPES oder Barbital-Acetat-Puffer aber auch andere Puffersysteme, bevorzug ist Tris.

Weiter bevorzugt ist es, wenn die Formulierungen oder Mundspüllösungen mindestens ein Puffersystem, vorzugsweise eine Puffersubstanz aus der Gruppe der primären Alkalicitrate, der sekundären Alkalicitrate und/oder einem Salz von Carbonsäuren, insbesondere mit 1 bis 20 C-Atomen, vorzugsweise ein Salz mindestens einer Fruchtsäure, ein Salz einer alpha-Hydroxysäure und/oder ein Salz einer Fettsäure, insbesondere mit 1 bis 20 C-Atomen. Besonders bevorzugte Salze der vorgenannten Säuren sind die Alkali-, Erdalkali- und/oder Zink-Salze der Citronensäure, Aepfelsäure, Weinsäure und/oder Milchsäure oder Tris. Besonders bevorzugte Salze umfassen die Kationen von Natrium, Kalium, Magnesium und/oder Zink der vorgenannten Carboxylate.

Darüber hinaus ist Gegenstand der Erfindung die Verwendung der Formulierungen oder eines Kits zur Abscheidung von kristallinem Fluorapatit, Fluorapatitkristallen, nadelförmigem Fluorapatit, zahnschmelzähnlicher Überzüge aus Apatit auf Zahnhartsubstanz, Apatitkristallen, Apatit auf Oberflächen, Fluorapatit, Hydroxylapatit, Fluor-und Hydroxylapatit, auf Zähnen, auf Zahnschmelz, zur morphologischen Veränderung von Zahnoberflächen, zum Verschliessen von Lumen oder Kanalstrukturen in Zähnen, in Lumen, auf Dentin, zum Verschliessen offener Dentintubuli, zur Abscheidung von Apatit auf Knochen, auf einer Knochenmatrix, einer Formulierung und/oder eines Formkörpers in Form einer Negativform zur Abscheidung von Apatit in Lumen oder Kavitäten eines Zahns, insbesondere behandelten Lumen eines Zahns. Ferner ist Gegenstand der Erfindung die Verwendung einer Formulierung oder eines Kits zur Abscheidung von Apatit auf Oberflächen, insbesondere von Fluorapatit, mit einer Schichtdicke von grösser gleich 1 µm, vorzugsweise grösser gleich 2 µm, innerhalb von 24 Stunden, innerhalb von zweimal 12 Stunden, vorzugsweise innerhalb von 16 Stunden, insbesondere bei Körpertemperatur, 37 °C im Mundmilieu, bei 37 °C und einer Luftfeuchtigkeit von 95 %. Bevorzugt braucht die erfindungsgemässe Formulierung nur ein- bis zweimal für je 4 bis 12 Stunden angewendet zu werden, um bereits eine im Wesentlichen flächendeckende, vorzugsweise kristalline Apatitabscheidung mit einer Schichtdicke von grösser gleich 2 µm, besser grösser gleich 4 bis grösser gleich 5 µm zu erhalten.

Verwendung einer Formulierung enthaltend mindestens ein gut wasserlösliches Calcium-Salz, wie vorstehend offenbart, optional einen Gehalt eines Puffersystems, optional einen Gehalt an Maskierungsmittel oder Aromatisierungsmittel sowie üblichen Formulierungshilfsmitteln zur Herstellung einer Vorbehandlungslösung synonym zu Mundspüllösung oder zusammen mit Wasser als Vorbehandlungslösung vor einer Behandlung in der Apatit auf Zähnen von Wirbeltieren abgeschieden wird.

Verwendung einer Formulierung enthaltend Phosphat oder zu wasserlöslichen Phosphat-Ionen hydrolysierbare Phosphate, wasserlösliche Fluoride oder eine Fluoride freisetzende Verbindung und wasserlösliche Calcium-Ionen oder Calcium-Ionen freisetzende Verbindungen für eine remineralisierende Behandlung von Zähnen durch ein- oder zweimalige orale Applikation der Formulierung des teilelastischen Formkörpers aufweisend zumindest eine teilweise Hülle, vorzugsweise eine im Wesentlichen vollständige Hülle, vor dem Schlafengehen und Verwendung der Formulierung über Nacht oder über Tag zur Bildung von insbesondere kristallinen Apatitablagerungen, Fluorapatit, insbesondere mit einer Schichtdicke nach einmaliger Anwendung von grösser gleich 2 µm, vorzugsweise grösser gleich 4 µm. Dabei sind für die Verwendung bereits folgende Formkörper mit einer sehr geringen Schichtdicke ausreichend: (I) Formkörper in Form eines Flächenelements oder Kiefernegativ mit innenliegenden Flächenelementen als Schichten, mit mindestens zwei optional durch eine Membran getrennte Mineralisationsmatrices enthaltend das Gel mit einer Schichtdicke von 50 bis 6000 µm, vorzugsweise 50 bis 750 µm, besonders bevorzugt 50 bis 600 µm, oder (II) zwei separate Formkörper mit mindestens jeweils einer Mineralisationsmatrix enthaltend Gel, wobei jeder Formkörper jeweils unabhängig eine Schichtdicke von 10 bis 3000 µm aufweist, wobei der erste Formkörper umfassend Phosphate oder zu wasserlöslichen Phosphat-Ionen hydrolysierbare Phosphate eine Schichtdicke von 50 bis 3000 µm und/oder der zweite Formkörper umfassend Calcium-Ionen oder Calcium-Ionen freisetzende Verbindungen eine Schichtdicke von 10 bis 3000 µm aufweist.

Die erfindungsgemässen Formulierungen können vorzugsweise zur Behandlung empfindlicher Zähne, empfindlicher Zahnhälse, säureerodierter Zähne, rissiger Zähne, oberflächliche abradierter Zähne, freifreiliegender Zahnhälse, gebleichter Zähne, Zähnen nach einer Behandlung kariöser Zahnbereiche ein- (once-a-day) bis zweifach (bspw. one-day-mineralisation) angewendet werden, um eine 2 bis grösser gleich 5 µm dicke, vorzugsweise homogene und im Wesentlichen kristalline Apatitschicht auf den behandelten Oberflächen auszubilden. Grundsätzlich kann die Formulierung bei Bedarf häufiger angewendet werden, beispielsweise nach definierten Zeiträumen.

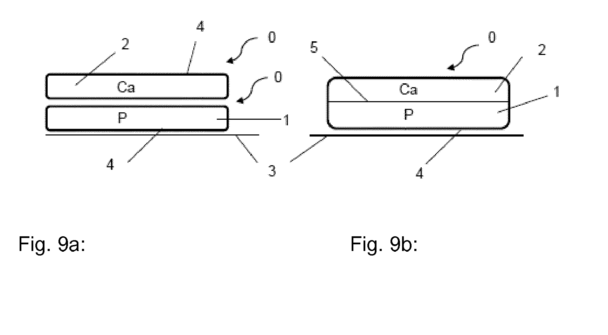

Die Figuren 9a und 9b offenbaren allgemeine Ausführungsformen der erfindungsgemässen Formkörper. Figur 9a zeigt zwei Formkörper 0, wobei die Mineralisationsmatrix 2 die Calcium-Komponente und die Mineralisationsmatrix 1 die Phosphat-Komponente umfasst. Die Hülle (Vernetzung, Beschichtung) wird dargestellt durch 4 und die optionale Membran(schicht) durch 5. In Figur 9a sind zwei Formkörper mit je einer Mineralisationsmatrix und in Figur 9b ein Formkörper mit zwei Mineralisationsmatrices in einer Hülle dargestellt. Die Mineralisationsmatrices können auch jeweils Calcium unterschiedlicher Konzentration enthalten bzw. entsprechend für Phosphat.

Die Erfindung wird anhand der nachfolgenden Beispiele und Figuren näher erläutert, ohne sie auf diese Beispiele zu beschränken.

**Beispiel 1 (Vergleichsbeispiel):** Für die phosphationenhaltige Komponente A wird eine Lösung hergestellt, die 29,5 g NaH₂PO₄, 33 g Olaflur, und 27,0 g Milchsäure enthält. Der pH-Wert wird mit 5 N Natronlauge auf 5,4 eingestellt und die Lösung wird mit entionisiertem Wasser auf 250 ml aufgefüllt. 24 ml der Lösung werden mit 6 g Glycerin und 10 g einer 300 Bloom Schweineschwarte-Gelatine unter Erwärmen zu einer dickflüssigen Lösung verarbeitet. In eine Schablone mit der Wandstärke von 500 µm wird etwas Flüssigkeit eingebracht und unter 2 bar Druck gepresst. Nach Verfestigung werden die Streifen der Schablone entnommen und in 1x1 cm grosse Quadrate geschnitten.

Für die calciumionenhaltige Komponente B wird eine Calciumchloridlösung angesetzt, die 29,4 g Calciumchlorid-Dihydrat und 6,3 g Milchsäure enthält. Mit 5 N Natronlauge wird ein pH-Wert von 4.0 eingestellt. Die Lösung wird mit entionisiertem Wasser auf 200 ml aufgefüllt. Zur Herstellung des Gels werden 21,6 g der Lösung mit 8,24 g Glycerin und 8 g Calciumsulfat und 13,6 g 300 Bloom-Gelatine vermischt und erwärmt. Das flüssige Gel wird mit einer Rakel auf eine Dicke von 1 mm ausgestrichen oder in einer Schablone mit der Wandstärke von 1 mm gepresst. Nach Verfestigung werden die Streifen werden sie 1x1 cm grosse Quadrate geschnitten. Für die Vorbehandlungslösung (Mundspüllösung) wird eine 1 molare Calciumchloridlösung mit 0.1 mol Tris-Puffer versetzt und auf den pH-Wert von 9,0 eingestellt.

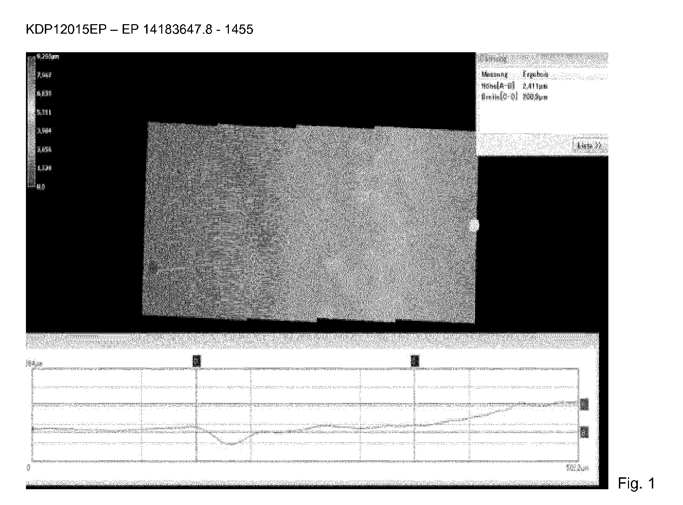

Zur Bewertung der Mineralisationsaktivität werden je 6 Zahnscheiben für 10 s mit 1 M HCl angeäzt, mit der Vorbehandlungslösung abgespült und mit je einem Stück Phosphat-Gel und einem Stück Calciumgel bedeckt. Um die morphologische Änderung der Zahnoberfläche deutlich zu machen, wurde eine Scheibe zuvor zur Hälfte abgeklebt, so dass sie nur hälftig remineralisieren kann. Die Proben werden im Klimaschrank bei 37°C und 95% Luftfeuchtigkeit aufbewahrt und nach 8 bis 16 Stunden mit lauwarmen Wasser und einer weichen Zahnbürste gereinigt. Nach nur einer Behandlung ist die Zahnoberfläche zum grössten Teil mit einer fest haftenden Schicht überzogen. Abbildung 1 zeigt die typische Oberflächenmorphologie der Beschichtung nach einer Behandlung im Grenzbereich zwischen beschichteter und unbeschichteter Probe. Die Schicht kann bis zu 2.5 µm dick sein. Die Gele waren nach 8 bis 16 Stunden auf den Proben verlaufen. Dieser Nachteil ist für den Nutzer nicht akzeptabel für eine Anwendung der Matrices im Mund.

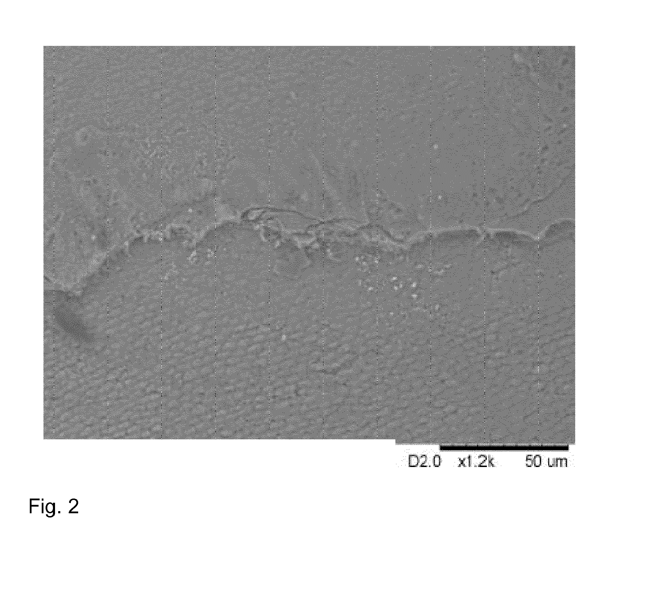

Figur 1: Typische Schichten nach einer Anwendung des Mineralisations-Kits 1: Lichtmikroskopische Aufnahme in Falschfarben, 3D-Mikroskop von Keyence, typische Schicht nach einer Anwendung des Mineralisations-Kits. Die Farben repräsentieren die unterschiedlichen Höhen. Aus dem Linescan lässt sich die Schichtdicke bestimmen. Links: unbehandeltes Dentin (blau) rechts: behandeltes Dentin (grün-rot). Die Kanalstruktur verschwindet unter einer dichten Schicht (3-D-Mikrsokop, Keyence), die Schicht kann bis zu 2.5 µm dick sein. Figur 2: REM-Aufnahme der Grenzfläche Schmelz beschichtet - unbeschichtet).

**Beispiel 2:** Die Herstellung der Komponenten erfolgt wie unter Beispiel 1 beschrieben, der pH-Wert der Phosphat-Komponente wird jedoch auf 5.6 eingestellt, der der Calcium-Komponente auf 4.3. Für die Phosphat-Komponente wird eine Vernetzungslösung hergestellt. Dazu werden 1.5 g 25%ige Glutardialdehydlösung mit der zuvor hergestellten Phosphat(P)-Lösung auf 100 g aufgefüllt. Zur Vernetzung werden die Gelplatten in 1cm²- grosse Quadrate geschnitten und auf eine Folie gesetzt. Im Anschluss werden sie für 20 s in der Vernetzerlösung gebadet. Dann werden sie mit der P-Lösung ohne Vernetzerkomponente abgewaschen und trocken geblasen. Für die Ca-Komponente wird eine Vernetzerlösung hergestellt. Dazu werden 2 g 25 Gew.-%ige Glutardialdehydlösung mit der zuvor hergestellten Ca-Lösung auf 100 g aufgefüllt. Zur Vernetzung werden die Streifen in 1cm²- grosse Quadrate geschnitten, auf die Folie gesetzt und für 40 s der Vernetzerlösung ausgesetzt. Im Anschluss werden sie mit der Ca-Lösung ohne Vernetzerkomponente abgewaschen und trocken geblasen.

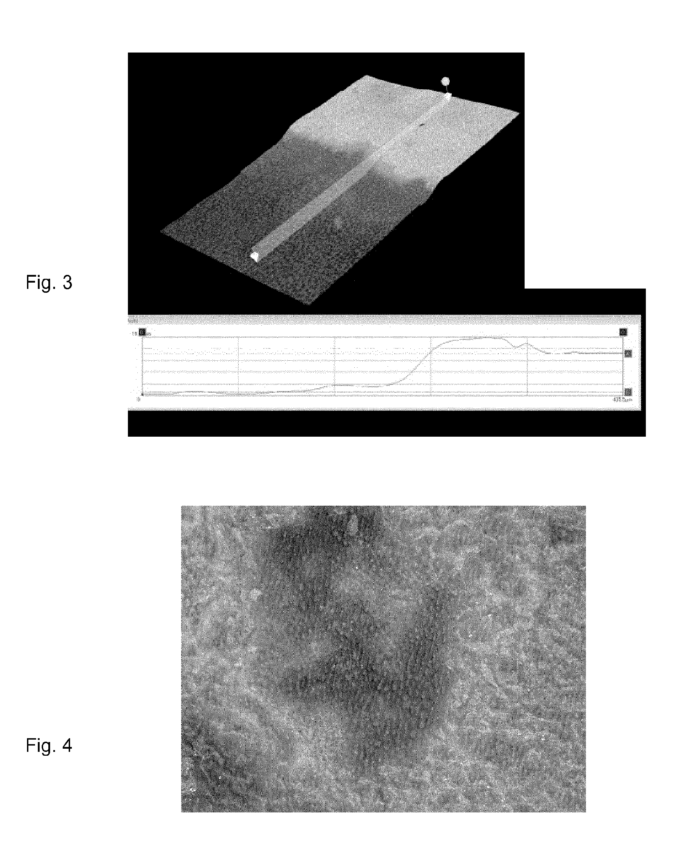

Zur Bewertung der Mineralisationsaktivität werden je 6 Zahnscheiben der Vorbehandlungslösung abgespült und mit je einem Stück Phosphat-Gel und einem Stück Kalziumgel bedeckt. Um die morphologische Änderung der Zahnoberfläche deutlich zu machen, wurde eine Scheibe zuvor zur Hälfte abgeklebt, so dass sie nur hälftig remineralisieren kann. Die Proben werden im Klimaschrank bei 37°C und 95% Luftfeuchtigkeit aufbewahrt und nach 12 Stunden mit lauwarmen Wasser und einer weichen Zahnbürste gereinigt. Nach nur einer Behandlung ist die Zahnoberfläche teilweise vollständig mit einer fest haftenden Schichtüberzogen. Abbildung 4 zeigt die typische Oberflächenmorphologie der Beschichtung nach einer Behandlung im Grenzbereich zwischen beschichteter und unbeschichteter Probe. Die Schicht kann an einzelnen Stellen insbesondere im Grenzbereich bis zu 7 µm bis 10 µm dick sein. Im Durchschnitt liegt die Schichtdicke bei 2 bis 3 µm. Um die Bereiche zwischen erfolgreicher Beschichtung und freiliegendem Dentin mit dem blossen Auge sichtbar zu machen, werden die Proben nach der Reinigung mit 0.1 Gew.-%iger Rhodaminlösung getränkt und kurz abgespült. Die porösen Dentinbereiche färben sich rosa-rot, während die überwachsenen Areale aufgrund Ihrer Dichtigkeit keine Farbe mehr annehmen. Damit lassen sich nicht erfolgreich beschichtete Bereiche gut erkennen (siehe Figuren 3 und 4).

Figur 3: Typische Schicht nach einer Anwendungen des Mineralisations-Kits und Anfärben mit 0.1%iger Rhodaminlösung. links: unbehandeltes Dentin dunkel (bzw. rot) rechts, hell behandeltes Dentin (weiss), insbesondere im Grenzbereich ist die Apatitschicht bis zu 7 µm bis 10 µm. Die Kanalstruktur verschwindet unter einer dichten Schicht. Abbildung. 4 Teilweise nicht erfolgreich beschichtete Bereiche können durch den Färbetest verdeutlicht werden (Fig.: 1, 3 und 4: 3-D-Mikroskop, Keyence).

**Beispiel 3:** Die Herstellung der Komponenten erfolgt wie unter Beispiel 1 beschrieben. Der pH-Wert der Ca-Lösung wird jedoch auf 4.01 eingestellt. Für die P-Komponente wird eine Vernetzungslösung hergestellt. Dazu werden 2 g 25%ige Glutardialdehydlösung mit der zuvor hergestellten P-Lösung auf 100 g aufgefüllt. Zur Vernetzung werden die Streifen in 2cm²- grosse Quadrate geschnitten und für 40 s in der Vernetzerlösung geschwenkt. Im Anschluss werden sie mit der P-Lösung ohne Vernetzerkomponente abgewaschen und trocken geblasen. Für die Ca-Komponente wird eine Vernetzerlösung hergestellt. Dazu werden 2 g 25 Gew.-%ige Glutardialdehydlösung mit der zuvor hergestellten Ca-Lösung auf 100g aufgefüllt. Zur Vernetzung werden die Streifen in 2 cm²-grosse Quadrate geschnitten und für 40 s in der Vernetzerlösung geschwenkt. Im Anschluss werden sie mit der Ca-Lösung ohne Vernetzerkomponente abgewaschen und trocken geblasen.

Zur Bewertung der Mineralisationsaktivität werden 2 ganze humane Frontzähne mit maximal 1 mm Abstand zueinander mit der Wurzel in einen Harz eingebettet wodurch man einen simulierten Interdentalraum erhält. Für diese Zahnreihe wird eine individualisierte Tiefziehschiene angefertigt die 1,5 mm Platz für die Gelkomponente lässt. Die dreidimensionalen Gelformen werden bei Bedarf etwas eingekürzt, mit je einem Stück Phosphat-Gel und einem Stück Calziumgel bedeckt. Um die morphologische Änderung der Zahnoberfläche deutlich zu machen, wurde eine Scheibe zuvor zur Hälfte abgeklebt, so dass sie nur hälftig remineralisieren kann. Die Proben werden im Klimaschrank bei 37°C und 95% Luftfeuchtigkeit aufbewahrt und nach 12 bis 16 Stunden mit lauwarmem Wasser und einer weichen Zahnbürste gereinigt. Nach nur einer Behandlung ist die Zahnoberfläche teilweise vollständig mit einer fest haftenden Schichtüberzogen.

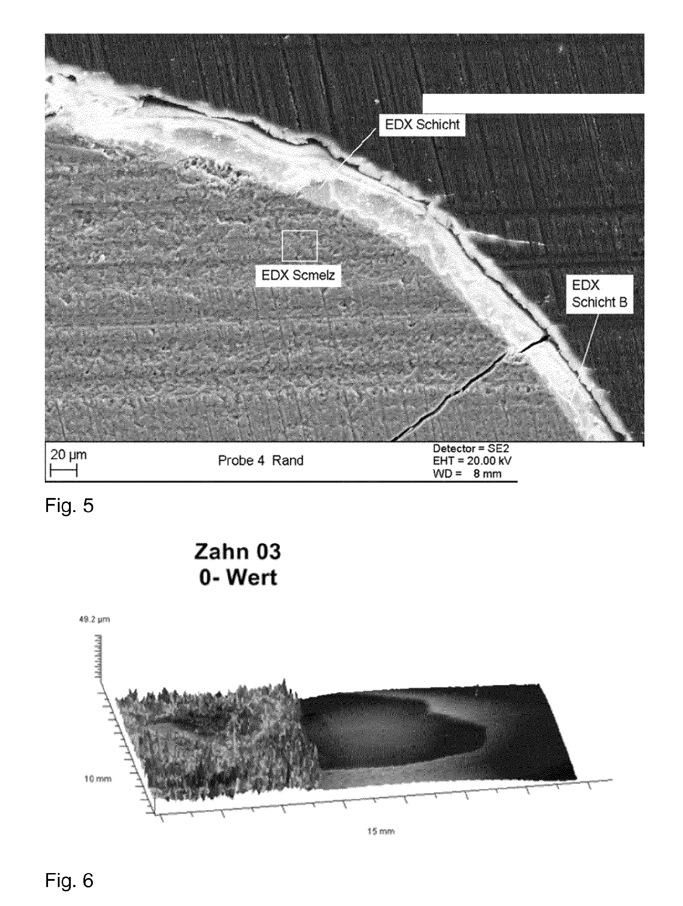

Zur Identifikation der Schicht mittels EDX-Analyse werden die Zähne quer gesägt und zwar in der Weise, dass die aufgewachsene Schicht im Querschnitt zu erkennen ist. Abbildung 5 zeigt einen Ausschnitt des oberen Zahnrandes mit der fluoridreichen Schicht. Die Analyse von Fluorid, Calcium und Phosphat wird als Nachweis für die Bildung eines fluoridhaltigen Calciumphosphates gewertet. In natürlichem Zahnschmelz lässt sich mittels EDX im Allgemeinen kein Fluorid nachweisen. Die gemessene Schichtdicke von weit über 20 µm kann nicht mit der tatsächlichen Schichtdicke von ca. 5 µm korreliert werden, da bedingt durch die Präparation die Schicht schräg aufgesägt wurde und dadurch im Querschnitt dicker erscheint als sie tatsächlich ist.

Figur 5: REM-Aufnahme eines quer zur Labialfläche aufgesägten humanen Zahns (Schmelz) nach 2 Behandlungen mit einem Mineralisations-Kit. Während eine Analyse im Schmelz als Hauptkomponenten nur Calcium und Phosphat zeigen, ist in der mineralisierten Schicht neben Calcium und Phosphat auch Fluorid zu erkennen, was als Indikator für den erzeugten, künstlichen Zahnschmelz gewertet wird. Die Schicht wird im Interdentalraum immer dünner.

Über die EDX-Analysen konnte die mineralisierte Schicht nachgewiesen werden und vom natürlichen Schmelz neben der veränderten Morphologie anhand ihres höheren Fluoridgehaltes bestimmt werden. Entsprechend der Zusammensetzung von Apatit finden sich im Wesentlichen die Elemente Ca, P, O und F. Die %-Angaben sind semiquantitativ, da die EDX-Einheit nicht auf einen Fluorapatitstandard kalibriert wurde. Es konnten etwa 5,55 % Fluor neben etwa 55,52 % Sauerstoff, 0,57 % Natrium, 12,39 Phosphor, 0,24 % Chlor und 15,34 % Calcium nachgewiesen werden.

**Beispiel 4a:** Zur Herstellung der P-Komponente werden für die P-Lösung 5,9 g Na₂HPO₄, 9,1g Milchsäure, 6,6 g Olaflur und 0.6 g 5 M NaOH auf 50 ml mit entionisiertem Wasser aufgefüllt. Die Herstellung des Gels erfolgt wie unter Beispiel 1 beschrieben. Die Calciumlösung für die Ca-Komponente wird durch Lösen in entionisiertem Wasser von 14,7 g CaCl₂, 3,15 g Milchsäure, 10 g 5 m Natronlauge in insgesamt 100 ml Lösung hergestellt. Die Herstellung des Gels entspricht der Beschreibung in Beispiel 1. Das Gleiche gilt für die Vorbehandlungslösung.

Zur Bewertung der Mineralisationsaktivität werden je 6 Zahnscheiben für 10 s mit 1 M HCl angeäzt, mit der Vorbehandlungslösung abgespült und mit je einem Stück Phosphat-Gel und einem Stück Calziumgel bedeckt. Um die morphologische Änderung der Zahnoberfläche deutlich zu machen, wurde eine Scheibe zuvor zur Hälfte abgeklebt, so dass sie nur hälftig remineralisieren kann. Die Proben werden im Klimaschrank bei 37°C und 95 % Luftfeuchtigkeit aufbewahrt und nach 8 bis 12 Stunden mit lauwarmen Wasser und einer weichen Zahnbürste gereinigt. Nach nur einer Behandlung ist die Zahnoberfläche zum größten Teil mit einer fest haftenden Schicht überzogen. Um die Bereiche zwischen erfolgreicher Beschichtung und freiliegendem Dentin mit dem blossen Auge sichtbar zu machen, werden die Proben nach der Reinigung mit 0.1%iger Rhodaminlösung getränkt und kurz abgespült. Mittels Farbmessgerät wird der Farbunterschied delta E zwischen der beschichteten und der unbeschichteten Seite bestimmt. Der Durchschnittswert liegt bei 52 (STAB 12).

**Beispiel 4b:** Die Herstellung der Komponenten des Kits entspricht Beispiel 4a, jedoch wird das P-Gel für 30 s mit einer 0.375%igen GDA-Lösung behandelt, die durch Mischen der P-Lösung mit der entsprechenden Menge GDA hergestellt wird. Die Gelstreifen werden im Anschluss nur trocken getupft. Die Ca-Gele werden mit einer 0.25%igen GDA-Lösung 30s lang behandelt und im Anschluss trocken getupft. Die Bewertung der Mineralisationsaktivität entspricht dem Beispiel 4a. Der Durchschnittswert für delta E liegt bei 63 (STAB 5, Fehlerangabe).

**Beispiel 5 (Vergleichsbeispiel):** Die Herstellung der Gele erfolgte analog dem Beispiel 1. Zur Bewertung der Mineralisationsaktivität wurden je 6 Zahnscheiben mit je einem Stück Phosphat-Gel und einem Stück Calciumgel bedeckt. Um die morphologische Änderung der Zahnoberfläche deutlich zu machen, wurde eine Scheibe zuvor zur Hälfte abgeklebt, so dass sie nur hälftig remineralisieren kann. Die Proben wurden im Klimaschrank bei 37°C und 95 % Luftfeuchtigkeit aufbewahrt, täglich gewaschen und einer erneuten Gelbehandlung unterzogen. Nach drei Behandlungen war die Zahnoberfläche so gut wie vollständig mit einer fest anhaftenden Schicht überzogen.

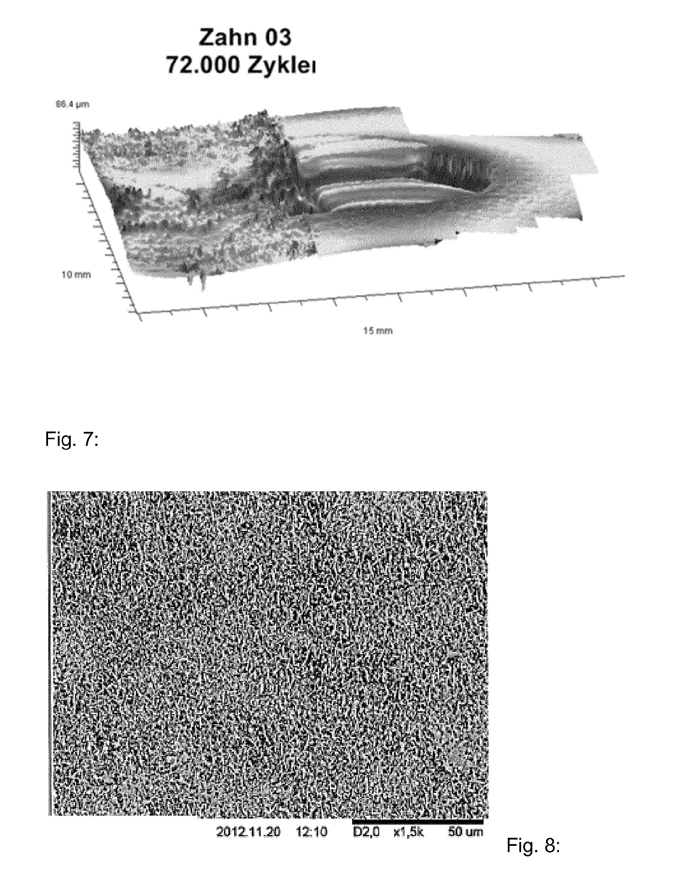

Die schmelzähnliche Stabilität lässt sich durch Zahnbürstabrasionsuntersuchungen zeigen. Nach 72.000 homogenen Bürstenstrichen (Belastung 150 g) auf einer Zahnprobe die halbseitig behandelt wurde, zeigt sich, dass das ungeschützte Dentin deutlich stärker abradiert wird als die ungeschützte Seite die ähnlich dem nativen Schmelz kaum abradiert wird. Figuren 6 und 7: 3x halbseitig behandelte Zahnscheiben vor der Zahnbürstabrasion (links) und nach der Zahnbürstabrasion. Es ist gut zu erkennen, dass die Behandlung mit dem Mineralisations-Kit das Dentin vor Abrasion schützt, das nicht behandelte Dentin wird stark abradiert., wie in einer 3D-Darstellung von biomimetisch behandelten Zähnen auf denen Fluorapatit abgeschieden und nicht behandelten Zähnen, deren Zahnflächen anschließend beide mittels Zahnbürste abradiert wurde. Die unbehandelten Zähne sind stark abradiert. Die Gele (ohne chemisch vernetze Ebene oder Hülle) waren nach jeder Behandlung auf den Proben verlaufen. Dieser Nachteil ist für den Nutzer nicht akzeptabel für eine Anwendung der Matrix im Mund.

**Beispiel 6:** Die Herstellung der Gele entspricht dem Beispiel 4b. Die Vernetzung wird aber bei beiden Gelen für jeweils 2x20s von beiden Seiten durchgeführt. Dabei liegt die GDA-Konzentration in der Ca-Vernetzerlösung bei 0.5 %. Die Bewertung der Mineralisationsaktivität entspricht den zuvor ausgeführten Beispielen. Im Elektronenmikroskop ist eine weitgehend homogene Schicht aus kleinen nadeligen Kristallen zu erkennen.

Fig. 8: Bewachsene Dentinoberfläche. Die porenreiche Dentinfläche ist von einer gleichmässigen Schicht nadeliger Kristalle überwachsen. Nach Behandlung mit dem Mineralisations-Kit (doppelseitige GDA-Vernetzung).

## Patentansprüche

1. Formulierung geeignet zur Abscheidung von Apatit ausgewählt aus Fluorapatit, Hydroxylapatit oder deren Gemischen auf Zähnen von Wirbeltieren, **dadurch gekennzeichnet, dass** die Formulierung mindestens einen teilelastischen Formkörper aufweist, umfassend mindestens eine Mineralisationsmatrix enthaltend mindestens ein Gel, wobei der Formkörper zumindest teilweise in mindestens einer Ebene, eine gegenüber wässrigen Medien verminderte Löslichkeit im Vergleich zur Mineralisationsmatrix aufweist, wobei die Ebene als Membran fungiert, und
a)die mindestens eine Mineralisationsmatrix ein Gel enthaltend wasserlösliche Phosphate oder zu wasserlöslichen Phosphat-Ionen hydrolysierbare Phosphate und einen pH-Wert von 2 bis 8 aufweist, und
b)die mindestens eine oder eine zweite Mineralisationsmatrix ein zweites Gel umfassend Calcium-Ionen oder Calcium-Ionen freisetzende Verbindungen und einen pH-Wert von 3.5 bis 14 aufweist.

2. Formulierung nach Anspruch 1, **dadurch gekennzeichnet, dass** die mindestens eine Mineralisationsmatrix in einem ersten Formkörper I. und die zweite Mineralisationsmatrix in einem zweiten Formkörper II. vorliegen.

3. Formulierung nach Anspruch 1 oder 2, **dadurch gekennzeichnet, dass** in
a) die mindestens eine Mineralisationsmatrix ein Gel aufweist, umfassend
(i) wasserlösliche Phosphate oder zu wasserlöslichen Phosphat-Ionen hydrolysierbare Phosphate,
(ii) einen Gehalt an Wasser oder ein Gemisch von Wasser und einem organischen Lösemittel,
(iii) optional mindestens eine Carbonsäure und/oder ein Puffersystem.

4. Formulierung nach Anspruch 1 oder 2, **dadurch gekennzeichnet, dass** die zweite Mineralisationsmatrix oder die mindestens eine Mineralisationsmatrix in (b) ein zweites Gel aufweist, umfassend
(i) Calcium-Ionen oder Calcium-Ionen freisetzende Verbindungen,
(ii) optional Wasser oder ein Gemisch von Wasser und einem organischen Lösemittel, und
(iii) optional mindestens eine Carbonsäure und/oder ein Puffersystem.

5. Formulierung nach einem der Ansprüche 1 bis 3, **dadurch gekennzeichnet, dass** die mindestens eine Mineralisationsmatrix ein Gel aufweist, umfassend mindestens ein wasserlösliches Fluorid oder eine Fluoride freisetzende Verbindung.

6. Formulierung nach einem der Ansprüche 1 bis 5, **dadurch gekennzeichnet, dass** das Gel mindestens einen Gelbilder ausgewählt aus denaturiertem Kollagen, Hydrokolloiden, Polypeptiden, Proteinhydrolysaten, Polysaccariden, Polyacrylaten oder Mischungen umfassend mindestens zwei der genannten Gelbilder enthält.

7. Formulierung nach einem der Ansprüche 1 bis 6, **dadurch gekennzeichnet, dass** das Gel Gelatine und ein Polyol, vorzugsweise Glycerin, deren Addukte und/oder deren Umsetzungsprodukte umfasst.

8. Formulierung nach einem der Ansprüche 1 bis 7, **dadurch gekennzeichnet, dass** der mindestens eine teilelastische Formkörper der mindestens einen Mineralisationsmatrix entspricht, wobei die Mineralisationsmatrix zumindest teilweise in mindestens einer chemisch mindestens teilvernetzten Ebene, eine gegenüber wässrigen Medien verminderte Löslichkeit als die entsprechende nicht auf diese Art chemisch vernetzte Mineralisationsmatrix aufweist.

9. Formulierung nach einem der Ansprüche 1 bis 8, **dadurch gekennzeichnet, dass** die mindestens eine Mineralisationsmatrix umfassend mindestens ein Gel in mindestens einem teilelastischen Formkörper in Form eines Flächenelements oder mindestens teilweisen Kiefernegativs vorliegt, wobei der Formkörper mindestens zwei an der äusseren Oberfläche angeordnete Ebenen oder zumindest eine teilweise äussere Hülle aufweist, die eine gegenüber wässrigen Medien verminderte Löslichkeit als die Mineralisationsmatrix aufweisen.

10. Formulierung nach einem der Ansprüche 1 bis 9, **dadurch gekennzeichnet, dass** die mindestens eine oder zwei Mineralisationsmatrices umfassend mindestens ein Gel in Form eines Flächenelements oder mindestens teilweisen Kiefernegativs vorliegen und/oder der mindestens eine teilelastische Formkörper in Form eines Flächenelements oder mindestens teilweisen Kiefernegativs vorliegt.

11. Formulierung nach einem der Ansprüche 1 bis 10, **dadurch gekennzeichnet, dass** die Ebenen oder die Hülle die äussere Begrenzung der Mineralisationsmatrix bilden.

12. Formulierung nach einem der Ansprüche 1 bis 11, **dadurch gekennzeichnet, dass** die mindestens eine Ebene oder Hülle gebildet wird durch eine chemische Vernetzung der Mineralisationsmatrix oder durch Auftragen einer Beschichtung auf die Mineralisationsmatrix zur Ausbildung einer Ionen und Wasser durchlässigen Oberfläche der Mineralisationsmatrix.

13. Formulierung nach einem der Ansprüche 1 bis 12, **dadurch gekennzeichnet, dass** die chemische Vernetzung in der mindestens einen Ebene oder zur Ausbildung der Hülle der Mineralisationsmatrix umfassend mindestens ein Gel basiert auf den chemischen Umsetzungsprodukten von Gelatine oder einer thermisch mit Glycerin stabilisierten Gelatine mit einem di- oder polyfunktionellen Vernetzer.

14. Formulierung nach einem der Ansprüche 1 bis 13, **dadurch gekennzeichnet, dass** sie umfasst:
(I) einen Formkörper in Form eines Flächenelements mit mindestens zwei getrennten Mineralisationsmatrices jeweils in Form eines Flächenelements enthaltend das Gel mit dem Schichtaufbau im Formkörper:
A) eine erste Mineralisationsmatrix in Form eines Flächenelements umfassend Gel und Phosphate oder zu wasserlöslichen Phosphat-Ionen hydrolysierbare Phosphate, wasserlösliche Fluoride oder Fluoride freisetzende Verbindung, Wasser oder ein Gemisch von Wasser und einem organischen Lösemittel, optional mindestens eine Carbonsäure und/oder ein Puffersystem,
B) optional Membran
C) eine zweite Mineralisationsmatrix in Form eines Flächenelements umfassend Gel und Calcium-Ionen oder Calcium-Ionen freisetzende Verbindungen, optional Wasser oder ein Gemisch von Wasser und einem organischen Lösemittel, optional mindestens eine Carbonsäure und/oder ein Puffer system,
wobei (I) der Formkörper in Form eines Flächenelements mindestens eine oder zwei an der äusseren Oberfläche angeordnete Ebenen oder eine äussere Hülle aufweist, die (a) zumindest teilweise durch chemische Vernetzung erhältlich sind oder (b) einer Beschichtung entsprechen, und die insbesondere als Membran fungieren, und die eine gegenüber wässrigen Medien verminderte Löslichkeit als die Mineralisationsmatrices aufweisen, oder
(II) zwei separate Formkörper jeweils unabhängig in Form eines Flächenelements mit mindestens jeweils einer Mineralisationsmatrix in Form eines Flächenelements enthaltend das Gel, und der erste Formkörper umfasst eine erste Mineralisationsmatrix in Form eines Flächenelements umfassend Gel und mindestens ein Phosphat oder zu wasserlöslichen Phosphat-Ionen hydrolysierbares Phosphat, und mindestens ein wasserlösliches Fluorid oder eine Fluoride freisetzende Verbindung, Wasser oder ein Gemisch von Wasser und einem organischen Lösemittel, optional mindestens eine Carbonsäure und/oder ein Puffersystem, und der
zweite Formkörper weist eine zweite Mineralisationsmatrix in Form eines Flächenelementes auf, umfassend Gel und mindestens eine Calcium-Ionen oder Calcium-Ionen freisetzende Verbindung, optional Wasser oder ein Gemisch von Wasser und einem organischen Lösemittel, optional mindestens eine Carbonsäure und/oder ein Puffersystem,
wobei (II) die Formkörper jeweils unabhängig mindestens eine oder zwei an der äusseren Oberfläche angeordnete Ebenen oder eine äussere Hülle aufweisen, die (a) zumindest teilweise durch chemische Vernetzung erhältlich sind oder
(b) einer Beschichtung entsprechen, und die insbesondere als Membran fungieren, und die eine gegenüber wässrigen Medien verminderte Löslichkeit als die Mineralisationsmatrices aufweisen.

15. Formulierung nach Anspruch 13 oder 14, **dadurch gekennzeichnet, dass** der di-oder polyfunktionelle Vernetzer umfasst Dialdehyde, Polyepoxide, und/oder Polyisocyanate sowie Mischungen umfassend mindestens zwei Vernetzer.

16. Formulierung nach einem der Ansprüche 1 bis 15, **dadurch gekennzeichnet, dass** die Carbonsäure ausgewählt ist aus Fruchtsäuren, Aminosäuren, Fettsäuren, Hydroxycarbonsäuren, Dicarbonsäuren und Mischungen umfassend mindestens zwei der genannten Säuren und/oder das Puffersystem umfasst Carboxylate von Alkylcarbonsäuren, Fettsäuren, Fruchtsäuren, Aminosäuren, Hydroxycarbonsäuren, Dicarbonsäuren und Mischungen umfassend mindestens zwei der genannten Säuren oder Phosphatpuffer.

17. Formulierung nach einem der Ansprüche 1 bis 16, **dadurch gekennzeichnet, dass** sie umfasst:
(I)einen Formkörper in Form eines Flächenelements mit mindestens zwei optional durch eine Membran getrennte Mineralisationsmatrices enthaltend das Gel mit einer Schichtdicke von 50 bis 6000 µm, oder
(II) zwei separate Formkörper jeweils unabhängig in Form eines Flächenelements mit mindestens jeweils einer Mineralisationsmatrix enthaltend ein Gel, wobei jeder Formkörper jeweils unabhängig eine Schichtdicke von 10 bis 3000 µm aufweist, wobei der erste Formkörper umfassend Phosphate oder zu wasserlöslichen Phosphat-Ionen hydrolysierbare Phosphate eine Schichtdicke von 50 bis 3000 µm und/oder der zweite Formkörper umfassend Calcium-Ionen oder Calcium-Ionen freisetzende Verbindungen eine Schichtdicke von 10 bis 3000 µm aufweist.

18. Verfahren zur Herstellung einer Formulierung, insbesondere nach einem der Ansprüche 1 bis 17, geeignet zur Abscheidung von Apatit ausgewählt aus Fluorapatit, Hydroxylapatit oder deren Gemischen auf Zähnen von Wirbeltieren indem,
(1) mindestens ein teilelastischer Formkörper umfassend mindestens eine Mineralisationsmatrix enthaltend mindestens ein Gel mit wasserlöslichen Phosphaten oder zu wasserlöslichen Phosphat-Ionen hydrolysierbare Phosphate hergestellt wird, wobei der Formkörper zumindest teilweise in mindestens einer Ebene, eine gegenüber wässrigen Medien verminderte Löslichkeit als die Mineralisationsmatrix aufweist, und der Formkörper hergestellt wird, indem
a)zur Herstellung mindestens einer Mineralisationsmatrix enthaltend das Gel in einem ersten Schritt eine Mischung von
(i) 0.05 bis 4 mol/l, insbesondere 0.5 bis 1.5 mol/l wasserlöslichen Phosphaten oder zu wasserlöslichen Phosphat-Ionen hydrolysierbaren Phosphaten,
(ii) einer entsprechenden Menge Wasser oder einem Gemisch von Wasser und einem organischen Lösemittel,
(iii) optional mindestens einer Carbonsäure und/oder eines Puffersystems
(iv) 0 bis 6000 Gew.-ppm wasserlöslichem Fluorid oder einer Fluoride freisetzenden Verbindung, gemischt werden,
in einem weiteren Schritt wird aus der in a) hergestellten Mischung
b)mit Gelatine und optional Glycerin unter Erwärmen das Gel hergestellt,
c) Formung des Gels unter Ausbildung der Mineralisationsmatrix, optional Verfestigung,
d)Ausbildung einer an der äusseren Oberfläche angeordneten Ebene der mindestens einen Mineralisationsmatrix unter Bildung des Formkörpers.

19. Verfahren zur Herstellung einer Formulierung, insbesondere nach einem der Ansprüche 1 bis 17, geeignet zur biomimetischen Abscheidung von Apatit ausgewählt aus Fluorapatit, Hydroxylapatit oder deren Gemischen auf Zähnen von Wirbeltieren indem,
(1) mindestens ein teilelastischer Formkörper umfassend mindestens eine Mineralisationsmatrix enthaltend mindestens ein Gel mit Calcium-Ionen oder Calcium-Ionen freisetzende Verbindungen hegestellt wird, wobei der Formkörper zumindest teilweise in mindestens einer Ebene, eine gegenüber wässrigen Medien verminderte Löslichkeit als die Mineralisationsmatrix aufweist, und der Formkörper hergestellt wird, indem
a)zur Herstellung mindestens einer Mineralisationsmatrix enthaltend das Gel in einem ersten Schritt eine Mischung von
(i) 0.1 bis 2 mol/l Calcium-Ionen oder Calcium-Ionen freisetzenden Verbindungen,
(ii) einer entsprechenden Menge Wasser oder einem Gemisch von Wasser und einem organischen Lösemittel,
(iii) optional mindestens einer Carbonsäure und/oder eines Puffersystems gemischt werden,
in einem weiteren Schritt wird aus der in a) hergestellten Mischung
b)mit Gelatine und optional Glycerin unter Erwärmen das Gel hergestellt,
c) Formung des Gels unter Ausbildung der Mineralisationsmatrix, optional Verfestigung,
d)Ausbildung einer an der äusseren Oberfläche angeordneten Ebene der mindestens einen Mineralisationsmatrix unter Bildung des Formkörpers.

20. Verfahren nach Anspruch 18 oder 19, **dadurch gekennzeichnet, dass** in b) im weiteren Schritt 5 bis 50 Gew.-% Gelatine, und 0 bis 30 Gew.-% Glycerin in Bezug auf die Gesamtzusammensetzung des Gels zugesetzt werden, bevorzugt werden 25 bis 40 Gew.-% Gelatine und 5 bis 20 Gew.-% Glycerin zur Herstellung der wasserlöslichen Phosphate oder zu wasserlöslichen Phosphat-Ionen hydrolysierbaren Phosphaten enthaltenden Matrix, und 20 bis 40 Gew.-% Gelatine und 15 bis 25 Gew.-% Glycerin zur Herstellung der Calcium-Ionen oder Calcium-Ionen freisetzende Verbindungen enthaltenden Mineralisationsmatrix zugesetzt.

21. Verfahren nach einem der Ansprüche 18 bis 20, **dadurch gekennzeichnet, dass** das im weiteren Schritt b) hergestellte Gel geformt wird, insbesondere zu einem Flächenelement oder einer individuellen dreidimensionalen Form, und das Gel in dieser Form verfestigbar ist.

22. Verfahren nach Anspruch 21, **dadurch gekennzeichnet, dass** mindestens eine an der äusseren Oberfläche angeordnete Ebene der mindestens einen Mineralisationsmatrix in Form eines Flächenelements oder mindestens teilweisen Kiefernegativs, im weiteren Schritt mit einer Mischung enthaltend einen di- oder polyfunktionellen Vernetzer umfassend Dialdehyde, Polyepoxide, Polyisocyanate, in Kontakt gebracht wird, und eine zumindest teilweise chemische Vernetzung in mindestens einer an der äusseren Oberfläche angeordneten Ebene des Flächenelements erzeugt wird, unter Erhalt des mindestens einen Formkörpers umfassend mindestens eine Ebene mit einer gegenüber wässrigen Medien verminderten Löslichkeit im Vergleich zur Mineralisationsmatrix, entsprechend einer Alternative werden alle an den äusseren Oberflächen angeordneten Ebenen des mindestens einen Flächenelementes vernetzt, um eine vernetzte Hülle der mindestens einen Mineralisationsmatrix herzustellen unter Erhalt des mindestens einen Formkörpers.

23. Verfahren nach einem der Ansprüche 18 bis 22, **dadurch gekennzeichnet, dass** mindestens eine Mineralisationsmatrix in Form eines Flächenelementes oder mindestens teilweisen Kiefernegativs zur Herstellung eines Phosphate und Fluoride enthaltenden Formkörpers unter Ausbildung einer zumindest teilweisen Hülle vernetzt wird und/oder mindestens eine Mineralisationsmatrix in Form eines Flächenelementes oder mindestens teilweisen Kiefernegativs zur Herstellung eines Calcium-Ionen enthaltenden Formkörpers unter Ausbildung einer zumindest teilweisen Hülle vernetzt wird.

24. Kit umfassend mindestens eine Formulierung aufweisend einen teilelastischen Formkörper A und einen separaten teilelastischen Formkörper B, jeweils unabhängig umfassend eine Formulierung nach einem der Ansprüche 1 bis 17 oder hergestellt nach einem der Ansprüche 18 bis 23, wobei
(a) der teilelastische Formkörper A umfasst
(a1) mindestens eine Mineralisationsmatrix umfassend mindestens ein Gel,
(a2) mindestens ein wasserlösliches Phosphat oder zu wasserlöslichen Phosphat-Ionen hydrolysierbare Phosphate, und
(a3) optional mindestens eine Carbonsäure und/oder ein Puffersystem
(a4) optional wasserlösliche Fluoride oder eine Fluoride freisetzende Verbindung
(a5) optional einen Gehalt an Wasser oder ein Gemisch von Wasser und einem organischen Lösemittel
(b) der teilelastische Formkörper B umfasst
(b1) mindestens eine Mineralisationsmatrix umfassend mindestens ein Gel,
(b2) wasserlösliche Calcium-Ionen oder Calcium-Ionen freisetzende Verbindungen, und
(b3) optional mindestens eine Carbonsäure und/oder ein Puffersystem
(b4) optional Wasser oder ein Gemisch von Wasser und einem organischen Lösemittel, oder
einen teilelastischen Formkörper C umfassend eine Formulierung, wobei der
(c) teilelastische Formkörper C umfasst
(c1) mindestens eine Mineralisationsmatrix umfassend mindestens ein Gel,
(c1.1) mindestens ein wasserlösliches Phosphat oder zu wasserlöslichen Phosphat-Ionen hydrolysierbares Phosphat, und
(c1.2) optional mindestens eine Carbonsäure und/oder ein Puffersystem
(c1.3) optional wasserlösliche Fluoride oder eine Fluoride freisetzende Verbindung
(c1.4) optional einen Gehalt an Wasser oder ein Gemisch von Wasser und einem organischen Lösemittel
(c2) optional eine Membran(schicht)
(c3) mindestens eine Mineralisationsmatrix umfassend mindestens ein Gel,
(c3.1) wasserlösliche Calcium-Ionen oder Calcium-Ionen freisetzende Verbindungen, und
(c3.2) optional mindestens eine Carbonsäure und/oder ein Puffersystem
(c3.3) optional Wasser oder ein Gemisch von Wasser und einem organischen Lösemittel, wobei der teilelastische Formkörper C den Schichtaufbau c1 und c3 oder c1, c2 und c3 aufweist.

25. Vernetzerlösung, insbesondere zur Verwendung bei der Herstellung einer Formulierung nach einem der Ansprüche 1 bis 17 oder 18 bis 23, **dadurch gekennzeichnet**, (a) eine Phosphat-Mischung hergestellt wird durch Mischen von
(i) 0.05 bis 4 mol/l, 0.5 bis 1.5 mol/l wasserlöslichen Phosphaten oder zu wasserlöslichen Phosphat-Ionen hydrolysierbaren Phosphaten,
(ii) einer entsprechenden Menge Wasser oder ein Gemisch von Wasser und einem organischen Lösemittel,
(iii) optional mindestens einer Carbonsäure und/oder eines Puffersystems
(iv) 0 bis 6000 Gew.-ppm wasserlöslichen Fluorids oder einer Fluorid freisetzenden Verbindung, oder
(b) eine Calcium-Mischung hergestellt wird durch Mischen von
(i) 0.1 bis 2 mol/l Calcium-Ionen oder Calcium-Ionen freisetzenden Verbindungen,
(ii) einer entsprechenden Menge Wasser oder einem Gemisch von Wasser und einem organischen Lösemittel,
(iii) optional mindestens einer Carbonsäure und/oder eines Puffersystems gemischt werden, und (a) und/oder (b) mit einer definierten Menge einer Lösung enthaltend einen di- oder polyfunktionellen Vernetzer umfassend Dialdehyde, Polyepoxide, Polyisocyanate, gemischt werden, insbesondere ist der Vernetzer Glutardialdehyd.

26. Verwendung einer Formulierung enthaltend Phosphat oder zu wasserlöslichen Phosphat-Ionen hydrolysierbare Phosphate, wasserlösliche Fluoride oder eine Fluoride freisetzende Verbindung und wasserlösliche Calcium-Ionen oder Calcium-Ionen freisetzende Verbindungen für eine remineralisierende Behandlung von Zähnen durch mindestens einmalige orale Applikation der Formulierung des mindestens teilelastischen Formkörpers mit zumindest einer teilweisen Hülle vor dem Schlafengehen und Verwendung der Formulierung über Nacht oder über Tag zur Bildung von kristallinen Apatitablagerungen, insbesondere mit einer Schichtdicke von teilweise grösser gleich 2 µm.
